(19) Europäisches Patentamt | European Patent Office | Office européen des brevets

(11) **EP 3 763 288 B1**

(12) **EUROPEAN PATENT SPECIFICATION**

(45) Date of publication and mention of the grant of the patent:
**19.06.2024 Bulletin 2024/25**

(21) Application number: **19764167.3**

(22) Date of filing: **05.03.2019**

(51) International Patent Classification (IPC):
**A61B 5/055** (2006.01)    **A61H 1/02** (2006.01)

(52) Cooperative Patent Classification (CPC):
**A61B 5/165; A61B 5/0042; A61B 5/055; A61B 5/486; G01R 33/4806**

(86) International application number:
**PCT/JP2019/008612**

(87) International publication number:
**WO 2019/172245 (12.09.2019 Gazette 2019/37)**

(54) **BRAIN ACTIVITY TRAINING DEVICE, BRAIN ACTIVITY TRAINING METHOD, AND BRAIN ACTIVITY TRAINING PROGRAM**

VORRICHTUNG, VERFAHREN UND PROGRAMM ZUM TRAINIEREN DER GEHIRNAKTIVITÄT

DISPOSITIF D'ENTRAÎNEMENT D'ACTIVITÉ CÉRÉBRALE, PROCÉDÉ D'ENTRAÎNEMENT D'ACTIVITÉ CÉRÉBRALE ET PROGRAMME D'ENTRAÎNEMENT D'ACTIVITÉ CÉRÉBRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2018 JP 2018042909**

(43) Date of publication of application:
**13.01.2021 Bulletin 2021/02**

(73) Proprietor: **Advanced Telecommunications Research Institute International**
**Soraku-gun**
**Kyoto 619-0288 (JP)**

(72) Inventors:
• **YAMADA, Takashi**
  **Soraku-gun, Kyoto 619-0288 (JP)**
• **KOCHIYAMA, Takanori**
  **Soraku-gun, Kyoto 619-0288 (JP)**
• **KAWATO, Mitsuo**
  **Soraku-gun, Kyoto 619-0288 (JP)**
• **YOSHIOKA, Toshinori**
  **Soraku-gun, Kyoto 619-0288 (JP)**

(74) Representative: **Müller Hoffmann & Partner Patentanwälte mbB**
**St.-Martin-Straße 58**
**81541 München (DE)**

(56) References cited:
**WO-A1-2014/178323      JP-A- 2013 173 035**
**JP-A- 2015 116 213      JP-A- 2019 063 478**
**US-A1- 2015 294 074**

• **TAKASHI YAMADA ET AL: "Resting-State Functional Connectivity-Based Biomarkers and Functional MRI-Based Neurofeedback for Psychiatric Disorders: A Challenge for Developing Theranostic Biomarkers", INTERNATIONAL JOURNAL OF NEUROPSYCHOPHARMACOLOGY, vol. 20, no. 10, 17 July 2017 (2017-07-17) , pages 769-781, XP055589968, Cambridge ISSN: 1461-1457, DOI: 10.1093/ijnp/pyx059**
• **KAWATO, MITSUO: "Brain dynamics and neuropsychiatric disorders", Japanese Journal of Neuropsychology, vol. 32, no. 4, December 2016 (2016-12), pages 264-275, XP055637851,**
• **YAMADA, TAKASHI: "Resting-State Functional Connectivity-Based Biomarkers and Functional MRI- Based Neurofeedback for Psychiatric Disorders: A Challenge for Developing Theranostic Biomarkers", International Journal of Neuropsychopharmacology, vol. 20, no. 10, 1 October 2017 (2017-10-01), pages 769-781, XP055589968, DOI: 10.1093/ijnp/pyx059**

- ICHIKAWA, NAHO et al: "Identifying melancholic depression biomarker using whole-brain functional connectivity", arxiv.org, 2017, XP055637860, Retrieved from the Internet: URL:https://arxiv.org/ftp/arxiv/papers/170 4/1704.01039.pdf [retrieved on 2019-05-20]
- FUKUDA, MEGUM I: "Real-time fMRI Neurofeedback Training Induced Change in Correlation of Neural Activity in an Imagery Task and Resting State", Library.naist.jp, 2011, pages 1-2, XP055727580, Retrieved from the Internet: URL:https://library.naist.jp:19943/dspace/ handle/10061/6234 [retrieved on 2019-05-20]
- SAIOTE, CATARINA: "Resting-state functional connectivity and motor imagery brain activation", Human Brain Mapping, vol. 37, no. 11, November 2016 (2016-11), pages 3847-3857, XP055637872,

**Description**

TECHNICAL FIELD

The present invention relates to a brain activity training apparatus and a brain activity training program, utilizing functional brain imaging.

BACKGROUND ART

(Biomarker)

When biological information is converted into a numerical value and quantified as an index for quantitatively comprehending biological changes in a living body, it is called a "biomarker."

According to FDA (United States Food and Drug Administration), a biomarker is regarded as "a characteristic that is objectively measured and evaluated as an indicator of normal biological processes, pathogenic processes or pharmacological responses to a therapeutic intervention." Biomarkers representative of a state of or a change in a disease, or a degree of healing are used as surrogate markers (substitute markers) to monitor efficacy in clinical tests of new drugs. The blood sugar level, the cholesterol level and the like, are representative biomarkers used as indexes of lifestyle diseases. Biomarkers include not only substances of biological origin contained in urine or blood but also electrocardiogram, blood pressure, PET images, bone density, lung function and the like. Developments in genomic analysis and proteome analysis have led to discoveries of various biomarkers related to DNA, RNA or biological protein.

Biomarkers are promising for measuring therapeutic effectiveness after the onset of a disease and, in addition, as routine preventive indexes, promising for disease prevention. Further, application of biomarkers to individualized medicine for selecting effective treatment avoiding side effects is expected.

In the field of neurological/mental disorders, however, though molecular markers and the like usable as objective indexes from a biochemical or molecular genetics viewpoint have been studied, it should in fairness be stated that they are still in a research phase.

Meanwhile, a disease determination system has been reported, which uses an NIRS (Near-InfraRed Spectroscopy) technique to classify mental disorders such as schizophrenia and depression based on features of hemoglobin signals measured by biological optical measurement (Patent Literature 1).

In this connection, as regards autism and depression, there have been reports of discriminator/classifier techniques that utilize machine learning techniques on functional connectivity of brain activities in the resting state among brain areas, which will be described later, to extract functional connectivity characteristics to each disease and thereby to enable classification between healthy individuals and patients based on the extracted functional connectivity (Patent Literatures 2 and 3, Non-Patent Literature 1).

(Real Time Neurofeedback)

Meanwhile, real time neurofeedback is studied as the possible therapy for a neurological/mental disorder.

Functional brain imaging, such as functional Magnetic Resonance Imaging (fMRI), which visualizes hemodynamic reaction related to human brain activities using Magnetic Resonance Imaging (MRI), has been used to specify an active region of a brain corresponding to a component of a brain function of interest, that is, to clarify cerebral localization of function, by detecting differences between those brain activities while responding to a sensory stimulus or performing a cognitive task, and those brain activities in a resting state or while performing a control task.

On the other hand, recently, the real time neurofeedback technique using functional brain imaging such as functional magnetic response imaging (fMRI) is reported (Non-Patent Literature 2).

A major technical problem to realize the real time neurofeedback is how to reduce the time of imaging. In order to reduce the time of imaging, Non-Patent Literatures 3 and 4 disclose a so-called "multi-band imaging."

The real time neurofeedback technique mentioned above has been attracting attention as a method of treating mental disorder.

Neurofeedback is one type of bio-feedback, in which a subject receives feedback about his/her brain activities in order to learn how to manage his/her brain activities.

By way of example, according to a report, when measurements of activities of anterior cingulate gyrus were fed back to patients on a real time basis as larger or smaller fire images, and the patients were instructed to make efforts to decrease the size of the fire, then improvement was attained both in real-time and long-term chronic pain of central origin (see Non-Patent Literature 5).

(Resting State fMRI)

Further, recent studies show that even when a subject is in the resting state, his/her brain works actively. Specifically, in the brain, there is a group of nerve cells that subside when the brain works actively and are excited vigorously when the brain is in the resting state. Anatomically, these cells mainly exist on the medial surfaces where left and right cerebral hemispheres are connected, namely, the medial surface of the frontal lobe, the posterior cingulate gyrus, the precuneus, the posterior portion of the parietal association area and the middle temporal gyrus. These regions representing baseline brain activities in the resting state are named Default Mode Network (DMN) and these regions work in synchronization as one network (see Non-Patent Literature 6).

An example of a difference in brain activities between those of a healthy individual and those of a patient with a mental disease is observed in brain activities in the default mode network. The default mode network refers to portions of one's brain that exhibit more positive brain activities when a subject is in the resting state than when the subject is performing a goal-oriented task. It has been reported that abnormality has been observed in the default mode networks of patients with mental disorders such as schizophrenia or Alzheimer's disease as compared with healthy individuals. By way of example, it is reported that in the brain of a schizophrenia patient, correlation of activities among the posterior cingulate cortex, which belongs to the default mode network, and parietal lateral cortex, medial prefrontal cortex or cerebellar cortex, is decreased in the resting state.

On the other hand, observed changes in correlations between activities among a plurality of brain regions caused, for example, by a difference in tasks are used to evaluate functional connectivity among these brain regions.

Specifically, the evaluation of functional connectivity in the resting state obtained by fMRI is called resting-state functional connectivity MRI (rs-fcMRI), which is utilized for clinical studies directed to various neurological/mental disorders.

As a premise of studying the relation between functional connectivity and neurofeedback, a case is reported in which the degree of activity of a single specific brain region (ROI: Region Of Interest) is fedback to a subject as a sound signal pitch (see Non-Patent Literature 7).

However, it is not necessarily clear how the default mode network relates to a specific disease, or how the correlations of functional connections among brain regions relate to the above-described neurofeedback.

(DecNef Method: Decoded Neurofeedback Method)

Recently, a new type neural feedback method referred to as decoded neural feedback method (DecNef method) is reported (Non-Patent Literature 8 and Patent Literature 5).

Human sensory and esthetic systems are ever-changing in accordance with the surrounding environment. Most of the changes occur in a certain early period of the human developmental stage, or the period referred to as a "critical period." Adults, however, still keep sufficient degree of plasticity of sensory and esthetic systems to adapt to significant changes in surrounding environment. By way of example, it is reported that adults subjected to a training using specific esthetic stimulus or exposed to specific esthetic stimulus have improved performance for the training task or improved sensitivity to the esthetic stimulus, and that such results of training were maintained for a few months to a few years (see, for example, Non-Patent Literature 9). Such a change is referred to as sensory learning, and it has been confirmed that such a change occurs in every sense organ, that is, in each of vision, audition, olfaction, gustation, and taction.

According to DecNef method, a stimulus as an object of learning is not directly applied to a subject while brain activities are detected and decoded and only the degree of approximation to a desired brain activity is fed back to the subject to enable "sensory learning."

(Nuclear Magnetic Resonance Imaging)

Nuclear Magnetic Resonance Imaging as described above will be briefly described in the following.

Conventionally, as a method of imaging cross-sections of the brain or the whole body of a living body, nuclear magnetic resonance imaging has been used, for example, for human clinical diagnostic imaging, which method utilizes nuclear magnetic resonance with atoms in the living body, particularly with atomic nuclei of hydrogen atoms.

As compared with "X-ray CT," which is a similar method of human tomographic imaging, characteristics of nuclear magnetic resonance imaging when applied to a human body, for example, are as follows:

(1) An image with the density distribution is obtained reflecting the distribution of hydrogen atoms and their signal relaxation time (reflecting strength of atomic bonding). Therefore, the shadings present different natures of tissues, making it easier to observe a difference in tissues;

(2) The magnetic field is not absorbed by bones. Therefore, a portion surrounded by a bone or bones (for example, inside one's skull, or spinal cord) can easily be observed; and

(3) Unlike X-rays, it is not harmful to human body and, hence, it has a wide range of possible applications.

Nuclear magnetic resonance imaging described above uses the magnetic property of hydrogen atomic nuclei (protons), which are most abundant in human cells and have the highest magnetism. Motion in a magnetic field of spin angular momentum associated with the magnetism of the hydrogen atomic nucleus is, classically, compared to the precession of a spinning top.

In the following, as a description of the background of the present invention, the principle of magnetic resonance will be summarized using the intuitive classical model.

The direction of spin angular momentum of a hydrogen atomic nucleus (the direction of the axis of the rotation of a spinning top) is random in an environment free of the magnetic field. When a static magnetic field is applied, however, the momentum is aligned with the line of the magnetic force.

In this state, when an oscillating magnetic field is superposed and the frequency of the oscillating magnetic field is the resonance frequency $f0 = \gamma B0/2\pi$ ($\gamma$: substance-specific coefficient) determined by the intensity of the static magnetic field, energy moves to the side of the atomic nuclei because of resonance, and the direction of the magnetic vector changes (precession increases). When the oscillating magnetic field is turned off in this state, the precession gradually returns to the direction in the static magnetic field with the tilt angle returning to the previous angle. By externally detecting this process by an antenna coil, an NMR (Nuclear Magnetic Resonance) signal can be obtained.

The resonance frequency f0 mentioned above of hydrogen atom is $42.6 \times B0$ (MHz) where B0 (T) represents the intensity of the static magnetic field.

Further, in nuclear magnetic resonance imaging, changes appearing in detected signals in accordance with changes in the blood stream make it possible to visualize an active portion of a brain activated in response to an external stimulus. Such a nuclear magnetic resonance imaging is specifically referred to as fMRI (functional MRI).

An fMRI uses a common MRI apparatus with additional hardware and software necessary for fMRI measurement.

The changes in the blood stream cause changes in the NMR signal intensity, since oxygenated hemoglobin and deoxygenated hemoglobin in the blood have magnetic properties different from each other. Hemoglobin is diamagnetic when oxygenated, and it does not have any influence on relaxation time of hydrogen atoms in the surrounding water. In contrast, hemoglobin is paramagnetic when deoxygenated, and it changes surrounding magnetic field. Therefore, when the brain receives any stimulus and local blood stream increases and oxygenated hemoglobin increases, the change can be detected by the MRI signals. The stimulus to a subject may include visual stimulus, audio stimulus, or performance of a prescribed task (see, for example, Patent Literature 2).

Note that, in the studies of the brain functions, brain activities are measured by measuring increase in the nuclear magnetic resonance signal (MRI signal) of hydrogen atoms corresponding to a phenomenon where the density of deoxygenated hemoglobin in red blood cells decrease in minute veins or capillary vessels (BOLD effect).

Particularly, in studies related to the human motor functions, brain activities are measured by the MRI apparatus as described above while a subject or subjects are performing some physical activity.

For human subjects, it is necessary to measure the brain functions in a non-invasive manner. In this aspect, a decoding technique enabling extraction of more detailed information from fMRI data has been developed. Specifically, a voxel-by-voxel brain activity analysis (volumetric pixel: voxel) of the brain by the fMRI enables the estimation of stimulus input and the state of recognition from spatial patterns of the brain activities. The DecNef method mentioned above applies such a decoding technique to tasks related to perceptual learning.

Further, studies have been made to improve treatment of mental disorder such as autism, by feeding back to the subject the determination results of functional connectivity among brain regions (Patent Literature 4, Non-Patent Literatures 10 and 11) Non-Patent Literature 11 describes the appliation of a machine-learning algorithm to rs-fcMRI data from 74 high-functioning adults with autism spectrum disorder (ASD) and 107 typically developed (TD) adults to extract functional connectivity features (FCs) associated with the diagnostic labels. Multiple of the extracted FCs were selected as targets in FC-neurofeedback training held over 4 to 5 successive days.

CITATION LIST

PATENT LITERATURE

PTL 1: WO-A1-2006/132313
PTL 2: WO-A1-2014/178323
PTL 3: WO-A1-2017/090590

PTL 4: WO-A1-2014-178322
PTL 5: US 2015/294074 A1

NON PATENT LITERATURE

NPL 1: Ichikawa N, Lisi G, Yahata N, Okada G, Takamura M, Yamada M, Suhara T, Hashimoto R, Yamada T, Yoshihara Y, Takahashi H, Kasai K, Kato N, Yamawaki S, Kawato M, Morimoto J, Okamoto Y (2017) Identifying melancholic depression biomarker using whole-brain functional connectivity. arXiv:1704.01039
NPL 2: Nikolaus Weiskopf, "Real-time fMRI and its application to neurofeedback", NeuroImage 62 (2012) 682-692
NPL 3: Katsutoshi MURATA, "Siemens sha-sei MR souchi no genjo to doukou" (Current Status and Trend of MR devices manufactured by Seimens AG), MEDICAL IMAGING TECHNOLOGY Vol.32 No.1 January 2014 pp.3-7
NPL 4: Feinberg DA, Moeller S, Smith SM et al., "Multiplexed echo planar imaging for sub-second whole brain FMRI and fast diffusion imaging.", PLoS ONE 2010; 5(12):e15710.
NPL 5: deCharms RC, Maeda F, Glover GH et al., "Control over brain activation and pain learned by using real-time functional MRI", Proc Natl Acad Sci USA 102(51), 18626-18631, 2005
NPL 6: Raichle ME, Macleod AM, Snyder AZ. et. al. "A default mode of brain function", Proc Natl Acad Sci USA 98(2), 676-682, 2001
NPL 7: Tal Harmelech, Son Preminger, Eliahu Wertman, and Rafael Malach, "The Day-After Effect: Long Term, Hebbian-Like Restructuring of Resting-State fMRI Patterns Induced by a Single Epoch of Cortical Activation", The Journal of Neuroscience, May 29, 2013, 33(22):9488-9497
NPL 8: Kazuhisa Shibata, Takeo Watanabe, Yuka Sasaki, Mitsuo Kawato, "Perceptual Learning Incepted by Decoded fMRI Neurofeedback Without Stimulus Presentation", SCIENCE VOL 334 9 DECEMBER 2011
NPL 9: T. Watanabe, J. E. Nanez Sr, S. Koyama, I.Mukai, J. Liederman and Y. Sasaki: Greater plasticity in lower-level than higher-level visual motion processing in a passive perceptual learning task. Nature Neuroscience, 5, 1003-1009, 2002.
NPL 10: Noriaki Yahata, Jun Morimoto, Ryuichiro Hashimoto, Giuseppe Lisi, Kazuhisa Shibata, Yuki Kawakubo, Hitoshi Kuwabara, Miho Kuroda, Takashi Yamada, Fukuda Megumi, Hiroshi Imamizu, Jose E. Nanez Sr, Hidehiko Takahashi, Yasumasa Okamoto, Kiyoto Kasai, Nobumasa Kato, Yuka Sasaki, Takeo Watanabe, & Mitsuo Kawato, "A small number of abnormal brain connections predicts adult autism spectrum disorder", NATURE COMMUNICATIONS | DOI: 10.1038/ncomms11254
NPL 11: Takashi Yamada, Ryu-ichiro Hashimoto, Noriaki Yahata, Naho Ichikawa, Yujiro Yoshihara, Yasumasa Okamoto, Nobumasa Kato, Hidehiko Takahashi, Mitsuo Kawato, "Resting-State Functional Connectivity-Based Biomarkers and Functional MRI-Based Neurofeedback for Psychiatric Disorders: A Challenge for Developing Theranostic Biomarkers", International Journal of Neuropsychopharmacology (2017) 20(10): 769-781

SUMMARY OF INVENTION

TECHNICAL PROBLEM

While some applications of the brain activity analysis by functional brain imaging such as functional Magnetic Resonance Imaging as described above and neurofeedback technique utilizing the same to treatment of neurological/mental disorders have come to be considered a possibility, practical applications are not yet established at present.

By way of example, when we consider applications to the treatment of neurological/mental disorders, the analysis of brain activities using functional brain imaging as the above-described biomarker is promising as a non-invasive functional marker, and applications to the development of diagnostic methods and to searching/identification of target molecules aiming toward drug discovery for realizing basic remedies are also expected.

By way of example, consider a mental disorder such as depression. A practical biomarker using genes is not yet established and, therefore, the development of therapeutic agents remains difficult, since it is difficult to determine the effects of medication.

On the other hand, it has been indicated that the results of a mental disease diagnosis can be predicted to some extent based on connections among brain areas derived from fMRI data in the resting state. If a biomarker that can predict the results of a mental disease diagnosis based on the connections among brain areas derived from fMRI data is realized, it would be possible to realize a system of treating neurological/mental disorders by combining it with the neurofeedback method.

Further, beyond such treatment of neurological/mental disorder, it would be desirable if autonomous training to attain a more desirable brain state becomes possible.

The neurofeedback method to date, however, does not necessarily make clear specifics as to how the brain activity characteristics are to be extracted and how the information for feedback is to be generated when brain activity training is executed or when such training is applied to actual disease or disorders.

The present invention was made to solve the above-described problems and its object is to provide a brain activity training apparatus and a brain activity training program, for enabling training to change correlation of connections among brain areas, by generating effective feedback information based on correlation of a specific connection of brain areas measured by functional brain imaging.

SOLUTION TO PROBLEM

The above problems are solved by the subject-matter of the independent claims.

According to an aspect, the present invention provides a brain activity training apparatus for executing a neurofeedback training, including: a storage device storing information for specifying, based on signals representing brain activities at a plurality of prescribed regions in a brain of each of a plurality of first and second subjects in a resting state time-sequentially measured in advance, a functional connectivity as an object of training from at least one functional connectivity selected by a feature selection for determining a state of a prescribed functional connectivity through machine learning, from functional connectivities among the plurality of prescribed regions; wherein the first subject belongs to a first group having a first pattern of time correlation of functional connectivity serving as a target pattern, and the second subject belongs to a group having a second pattern of time correlation of functional connectivity different from the target pattern; the apparatus further including: a brain activity detecting device for time-sequentially detecting signals representing brain activities at a plurality of brain regions in a brain of a trainee of the neurofeedback training, the plurality of brain regions in the brain of the trainee corresponding to respective ones of the plurality of prescribed regions in the brains of the first and second subjects; a presenting device; and a processing device; wherein the neurofeedback training is done for a number of days and involves repetition of a plurality of trials, each trial including a resting period, a self-regulation period following the resting period and a presenting period in which feedback information is presented, following the self-regulation period; the processing device is configured such that: i) from signals detected from the trainee in the self-regulation period in a first day of said number of days by the brain activity detecting device, a baseline level of degree of activity of each of the prescribed regions corresponding to the functional connectivity as the object of training is calculated; ii) for the trainee, from the signals detected in the self-regulation period in said number of days other than the first day by the brain activity detecting device and from the baseline level, time correlation of degrees of activity of the prescribed regions corresponding to the functional connectivity as the object of training is calculated; iii) based on the calculated time correlation, a reward value is calculated in accordance with a degree of approximation to the target pattern; and iv) feedback information indicating magnitude of the reward value is presented by the presenting device to the trainee.

Preferably, the degree of approximation is defined by such a function that the degree of approximation becomes higher as the time correlation of the self-regulation period becomes closer to a target time correlation.

Preferably, the plurality of prescribed regions in the brains of the first and second subjects are anatomically defined brain areas; and the plurality of brain regions in the brain of the trainee corresponding to the functional connectivity as the object of training are regions specified in advance for each trainee as regions of which brain activity is activated or inactivated when a known task is executed for specifying the brain areas.

Preferably, the functional connectivity as the object of training include a functional connectivity between left Dorsolateral Prefrontal Cortex and left Precuneus and left posterior cingulated cortex.

Preferably, the first subject is a healthy person; and the second subject is a subject exhibiting symptoms of depression, and the symptoms of depression are those of melancholic depression.

According to another aspect, the present invention provides a computer program controlling the brain activity training apparatus described above.

Also disclosed is the use of the brain activity apparatus of the present invention according to the following brain activity training method of executing a neurofeedback training, by controlling a brain activity detecting device having a presentation device by a computer having a processor and a storage device, including: the step of storing information for specifying, based on signals representing brain activities at a plurality of prescribed regions in a brain of each of a plurality of first and second subjects in a resting state time-sequentially measured in advance, a functional connectivity as an object of training from at least one functional connectivity selected by a feature selection for determining a state of a prescribed functional connectivity through machine learning, from functional connectivities among the plurality of prescribed regions; wherein the first subject belongs to a first group having a first pattern of time correlation of functional connectivity serving as a target pattern, and the second subject belongs to a group having a second pattern of time correlation of functional connectivity different from the target pattern; the apparatus further including: a brain activity detecting device for time-sequentially detecting signals representing brain activities at a plurality of brain regions in a brain of a trainee of the neurofeedback training, the plurality of brain regions in the brain of the trainee corresponding to respective ones of the plurality of prescribed regions in the brains of the first and second subjects; the neurofeedback training is done for a number of days and involves repetition of a plurality of trials, each trial including a resting period, a self-regulation period following the resting period and a presenting period in which feedback information is presented, following the self-regulation period; the method further including: the step of the processor calculating for the trainee, from signals detected from the trainee in the self-regulation period in a first day of said number of days by the brain activity detecting device, a baseline level of degree of activity of each of the prescribed regions corresponding to the functional connectivity as the object of training; the step of the processor calculating for the trainee, from the signals detected in the self-regulation period in said number of days other than the first day by the brain activity detecting device and from the baseline level, time correlation of degrees of activity of the prescribed regions corresponding to the functional connectivity as the object of training; the step of the processor calculating, based on the calculated time correlation, a reward value in accordance with a degree of approximation to the target pattern; and the step of the processor presenting, by the presenting device, feedback information indicating the magnitude of the reward value to the trainee.

Preferably, the degree of approximation is defined by such a function that the degree of approximation becomes higher as the time correlation of the self-regulation period becomes closer to a target time correlation.

Preferably, the plurality of prescribed regions in the brains of the first and second subjects are anatomically defined brain areas; and the plurality of brain regions in the brain of the trainee corresponding to the functional connectivity as the object of training are regions specified in advance for each trainee as regions of which brain activity is activated or inactivated when a known task is executed for specifying the brain areas.

Preferably, the functional connectivity as the object of training includes a functional connectivity between left Dorsolateral Prefrontal Cortex and left Precuneus and left posterior cingulated cortex.

ADVANTAGEOUS EFFECTS OF INVENTION

According to the present invention, it is possible to change by training correlation of connections among brain areas by generating effective feedback information based on the correlation of a specific connection of brain areas measured by functional brain imaging.

BRIEF DESCRIPTION OF DRAWINGS

Fig. 1 is a schematic diagram showing an overall configuration of an MRI apparatus 10.
Fig. 2 is a hardware block diagram of data processing unit 32.
Fig. 3 shows regions of interest (ROIs) of a brain imaged by rs-fcMRI in accordance with an embodiment.
Fig. 4 shows a concept of correlation of changes over time in activities of brain areas in a resting state, or functional connectivity among brain areas in the resting state.
Fig. 5 shows a concept of a procedure for extracting a correlation matrix representing correlations of functional connections of ROIs in the resting state.
Fig. 6 shows a concept of a process for generating a discriminator serving as a biomarker, from the correlation matrix.
Fig. 7 is a functional block diagram for a discriminator generating process and a discriminating process by the generated discriminator.
Fig. 8 is a flowchart representing a process executed by data processing unit 32 for generating the discriminator serving as the biomarker.
Fig. 9 shows a functional connectivity extracted to discriminate a state indicating symptoms of depression.
Fig. 10 shows a concept of the process executed by the brain activity training apparatus.
Fig. 11 shows an example of a diagram presenting information to be fed back to a subject.
Fig. 12 shows an example of a neurofeedback training sequence.
Fig. 13 shows results of imaging by single-band imaging as compared with multi-band imaging.
Fig. 14 shows relations between a region of interest (ROI) of anatomical brain areas and regions activated with respect to a specific function.
Fig. 15 shows a working memory task and a relation between activation/inactivation of brain regions.
Fig. 16 is an illustration showing details of the neurofeedback training sequence.
Fig. 17 shows changes over time in brain activities in brain regions FMR1 and FMR2 in a trial period.
Fig. 18 shows changes over time in brain activities in brain regions FMR1 and FMR2 in a trial period.
Fig. 19 shows changes over time in actually measured brain activity signal indicating functional connectivity of a subject.
Fig. 20 shows changes over time in actually measured brain activity signal indicating functional connectivity of a subject.

Fig. 21 shows changes over time in actually measured brain activity signal indicating functional connectivity of a subject.

Fig. 22 shows changes over time in actually measured brain activity signal indicating functional connectivity of a subject.

DESCRIPTION OF EMBODIMENTS

(Definition of Terms)

In the present invention, "symptoms of depression" include at least one selected from: depressed mode; loss of interest or motivation; loss of energy; irritability; inhibition; decreased thinking power, concentration or decisiveness; feeling of worthlessness or guilt; thoughts of death or suicide or suicide attempt; morbid contents of thought; delusion; physical symptoms (including general malaise, headache, heaviness of head, body aches such as low back pain; palpitation, shortness of breath, decreased appetite or weight); and sleep disturbance. Preferably, the above-described symptoms of depression include symptoms related to Major Depressive Disorder (MDD) in accordance with Diagnostic and Statistical Manual of Mental Disorders (DSM) - IV.

In the present invention, preferably, depression is MDD, though not limited thereto as long as it involves the symptoms of depression described above. MDD includes melancholic MDD, non-melancholic MDD and treatment-resistant MDD. In the present specification, MDD may simply be referred to as depression.

For evaluating severity of depression, self-report Beck Depression Inventory has been used as supplemental information for screening or interview at the time of diagnosis.

Further, Hamilton Depression Rating Scale (HDRS) used by physicians as measures for evaluation is a multi-item questionnaire presenting indexes related to depression, serving as indexes for evaluating the degree of recovery. It is abbreviated as HAM-D.

In the present invention, subjects include those having the symptoms of depression described above, though not limited thereto. There is no age or sex limit. The subjects may or may not be in treatment for relieving the symptoms.

There is no concept of "cure" in treating depression and, hence, that the depression symptoms are relieved means that the symptoms are alleviated as compared with the past state, or that the symptoms are considered "remitted" from clinical findings.

[Embodiment 1]

In the following, a configuration of an MRI system in accordance with embodiments of the present invention will be described with reference to the drawings. In the embodiments below, components or process steps denoted by the same reference characters are the same or corresponding components or steps and, therefore, description thereof will not be repeated unless necessary.

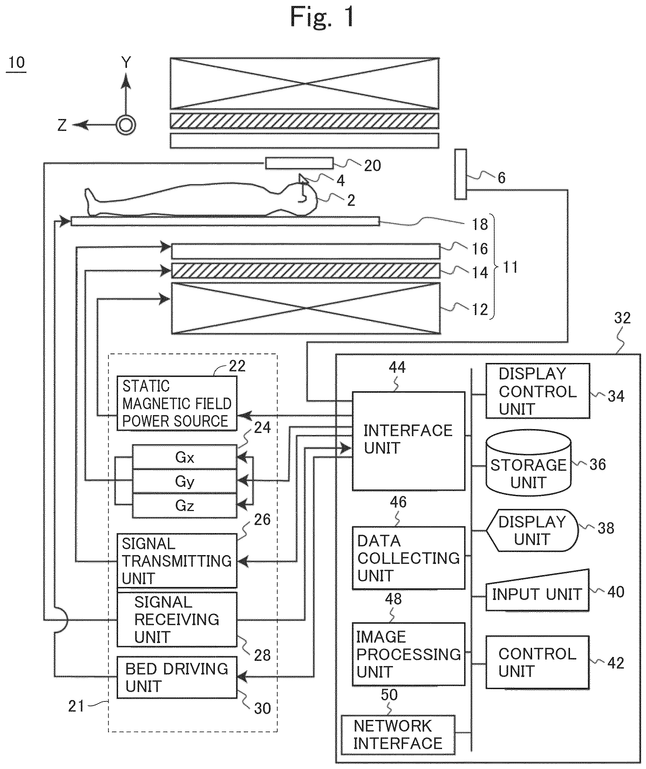

Fig. 1 is a schematic diagram showing an overall configuration of an MRI apparatus 10.

Referring to Fig. 1, MRI apparatus 10 includes: a magnetic field applying mechanism 11 applying a controlled magnetic field to, and irradiating with RF wave, a region of interest of a subject 2; a receiving coil 20 receiving a response wave (NMR signal) from subject 2 and outputting an analog signal; a driving unit 21 controlling the magnetic field applied to subject 2 and controlling the transmission/reception of RF wave; and a data processing unit 32 configuring a control sequence of driving unit 21 and processing various data signals to generate images.

Here, the central axis of a cylindrical bore in which subject 2 is placed is regarded as a Z-axis, and a horizontal direction orthogonal to the Z-axis and the vertical direction orthogonal to the Z-axis are defined as X-axis and Y-axis, respectively. In MRI apparatus 10 having such a configuration, because of the static magnetic field applied by magnetic field applying mechanism 11, nuclear spins of the atomic nuclei forming subject 2 are oriented in the direction of the magnetic field (Z-axis) and precess about the direction of the magnetic field, with the Larmor frequency unique to the atomic nuclei.

When irradiated with an RF pulse of the same Larmor frequency, the atoms resonate, absorb energy and are excited, resulting in nuclear magnetic resonance (NMR). When the irradiation with RF pulse is stopped after the resonance, the atoms discharge energy and return to the original, steady state. This process is referred to as a relaxation process. In the relaxation process, the atoms output electromagnetic wave (NMR signal) having the same frequency as the Larmor frequency.

The output NMR signal is received by receiving coil 20 as a response wave from subject 2, and the regions of interest of subject 2 are imaged by data processing unit 32.

Magnetic field applying mechanism 11 includes a static magnetic field generating coil 12, a magnetic field gradient generating coil 14, an RF irradiating unit 16, and a bed 18 having the bore wherein subject 2 is placed.

By way of example, subject 2 lies on his/her back on bed 18. Though not limited, subject 2 may view an image displayed on a display 6 mounted vertical to the Z-axis, using prism glasses 4. Visual stimulus is applied to subject 2 by an image on display 6. Alternatively, visual stimulus to subject 2 may be applied by projecting an image in front of subject 2 using a projector.

Such a visual stimulus corresponds to the presentation of feedback information in the above-described neurofeedback.

Driving unit 21 includes a static magnetic field power source 22, a magnetic field gradient power source 24, a signal transmitting unit 26, a signal receiving unit 28, and a bed driving unit 30 for moving bed 18 to any position along the Z-axis.

Data processing unit 32 includes: an input unit 40 for receiving the various operations and information input from an operator (not shown); a display unit 38 for displaying the various images and various pieces of information related to the regions of interest of subject 2, on a screen; a display control unit 34 for controlling the display of display unit 38; a storage unit 36 for storing programs, control parameters, image data (structural images and the like) and other electronic data to cause execution of the various processes; a control unit 42 controlling operations of various functional units, including generating a control sequence for driving the driving unit 21; an interface unit 44 for executing transmission/reception of various signals to/from driving unit 21; a data collecting unit 46 for collecting data consisting of a group of NMR signals derived from the regions of interest; an image processing unit 48 for forming images based on the data of NMR signals; and a network interface 50 for executing communication with a network.

Data processing unit 32 may be a dedicated computer, or it may be a general purpose computer executing functions causing operations of various functional units, in which designated operations, data processing and generation of control sequences are realized by a program or programs stored in storage unit 36. In the following, description will be given assuming that data processing unit 32 is implemented by a general purpose computer.

Static magnetic field generating coil 12 causes a current supplied from a static magnetic field power source 22 to flow through a helical coil wound around the Z-axis to generate an induction magnetic field, and thereby generates a static magnetic field in the Z-direction in the bore. The regions of interest of subject 2 are placed in the region of a highly uniform static magnetic field formed in the bore. More specifically, here, static magnetic field generating coil 12 is comprised of four air core coils, forms a uniform magnetic field inside by the combination of the coils, and attains orientation of the spins of the prescribed atomic nuclei in the body of subject 2, or more specifically, the spins of the hydrogen atomic nuclei.

Magnetic field gradient generating coil 14 is formed of X-, Y- and Z-coils (not shown), and provided on an inner peripheral surface of cylindrical static magnetic field generating coil 12.

These X-, Y- and Z- coils superpose magnetic field gradients on the uniform magnetic field in the bore with the X-axis, Y-axis and Z-axis directions switched in turn, whereby creating intensity gradient in the static magnetic field. When excited, the Z-coil tilts the magnetic field intensity to the Z-direction and thereby defines a resonance surface; the Y-coil applies a tilt for a short period of time immediately after the application of the magnetic field in the Z-direction, and thereby adds phase modulation in proportion to the Y-coordinate, to the detected signal (phase encoding); and thereafter the X-coil applies a tilt when data is collected, and thereby adds frequency modulation in proportion to the X-coordinate, to the detected signal (frequency encoding).

The switching of superposed magnetic field gradients is realized as different pulse signals are output to the X-, Y- and Z-coils from the magnetic field gradient power source 24 in accordance with a control sequence. Thus, the position of subject 2 expressed by the NMR can be specified, and positional information in three-dimensional coordinates necessary to form an image of subject 2 are provided.

Here, using the orthogonally crossing three sets of the magnetic field gradients, allocating slice direction, phase encoding direction and frequency encoding direction to the magnetic fields respectively, as described above, and by combining these, images can be taken from various angles. By way of example, in addition to transverse slice in the same direction as taken by an X-ray CT apparatus, saggital and coronal slices orthogonal thereto, as well as an oblique slice, of which direction vertical to its plane is not parallel to any of the axes of three orthogonally crossing magnetic field gradients, can be imaged.

RF irradiating unit 16 irradiates regions of interest of subject 2 with RF (Radio Frequency) pulses based on a high-frequency signal transmitted from a signal transmitting unit 26 in accordance with a control sequence.

Though RF irradiating unit 16 is built in a magnetic field applying mechanism 11 in Fig. 1, it may be mounted on bed 18 or integrated with receiving coil 20.

In MRI apparatus 10, in order to realize "multiband imaging" as disclosed in Non-Patent Literatures 3 and 4, RF irradiating unit 16 irradiates subject 2 with signals having RF pulses corresponding to a plurality of slices superposed, under the control of control unit 42.

Receiving coil 20 detects a response wave (NMR signal) from subject 2, and in order to detect the NMR signal with high sensitivity, it is arranged close to subject 2.

Here, when an electromagnetic wave of NMR signal crosses a coil strand of receiving coil 20, a weak current is generated by electromagnetic induction. The weak current is amplified by signal receiving unit 28 and converted from an analog signal to a digital signal, and then transmitted to data processing unit 32.

The mechanism here is as follows. To a subject 2 in a state of static magnetic field with a Z-axis magnetic field gradient added, a high-frequency electromagnetic field of resonance frequency is applied through RF irradiating unit 16. Prescribed atomic nuclei, for example, hydrogen atomic nuclei, at a portion where the magnetic field intensity satisfies the condition of resonance are selectively excited and start resonating. Prescribed atomic nuclei at a portion satisfying the condition of resonance (for example, a slice of prescribed thickness of subject 2) are excited, and spin axes of atomic nuclei concurrently start precession. When the excitation pulse is stopped, electromagnetic waves irradiated by the atomic nuclei in precession induce a signal in receiving coil 20 and, for some time, this signal is continuously detected. By this signal, a tissue containing the prescribed atoms in the body of subject 2 is monitored. In order to know the position where the signal comes from, X- and Y-magnetic field gradients are added and the signal is detected.

As described above, in order to perform multiband imaging (though not limiting, "multiband EPI imaging"), simultaneous multi-slice excitation is conducted on subject 2 by using signals having RF pulses of a plurality of frequencies (multiband) corresponding to a plurality of slices.

Based on the data built in storage unit 36, image processing unit 48 measures detected signals while repeatedly applying excitation signals, reduces resonance frequency to X-coordinate by a first Fourier transform to obtain an image, restores Y-coordinate by a second Fourier transform, and thus, displays the corresponding image on display unit 38.

In image processing unit 48, images of multiple slices, which have been collected simultaneously based on simultaneously excited and received signals for multiple slices and superposed over multiple slices, are separated by using parallel imaging technique (slice-GRAPPA: Slice-generalized auto-calibrating partially parallel acquisitions). Slice-GRAPPA technique is disclosed, for example, in the following reference.

Reference 1: Setsompop K, Gagoski BA, Polomeni JR, et.al.: "Blipped-controlled aliasing in parallel imaging for simultaneous multislice echo planar imaging with reduced g-factor penalty," Magn Reson Med 67:1210-1224, 2012.

In slice-GRAPPA, from a single band data and a virtual multiband data formed from a single band Reference Scan, a slice-GRAPPA kernel is formed, and superposed multiband EPI images are separated. While simply separating superposed images is disadvantageous from the viewpoint of g-factor and leakage factor, separation accuracy is improved by shifting, in a phase direction, the phase-modulated images of slices excited simultaneously at the time of imaging. Because of these features, the slice-GRAPPA technique successfully prevents SNR degradation even when multi-slices are simultaneously excited and collected.

For example, by picking-up the BOLD signals described above on a real-time basis using the MRI system as described above and performing an analysis on the images picked up in a time-sequential by control unit 42, it is possible to take images of the resting-state functional connection MRI (rs-fcMRI).

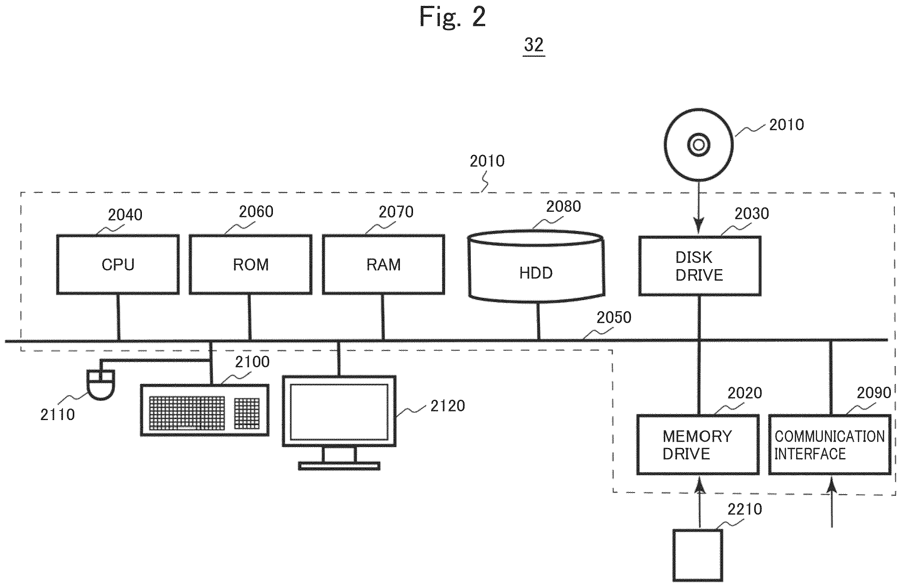

Fig. 2 is a hardware block diagram of data processing unit 32.

Though the hardware of data processing unit 32 is not specifically limited as described above, a general-purpose computer may be used.

Referring to Fig. 2, a computer main body 2010 of data processing unit 32 includes, in addition to a memory drive 2020 and a disk drive 2030, a processor (CPU) 2040, a bus 2050 connected to disk drive 2030 and memory drive 2020, an ROM 2060 for storing programs such as a boot-up program, an RAM 2070 for temporarily storing instructions of an application program and providing a temporary memory space, a non-volatile storage device 2080 for storing application programs, system programs and data, and a communication interface 2090. Communication interface 2090 corresponds to an interface unit 44 for transmitting/receiving signals to/from driving unit 21 and the like and a network interface 50 for communicating with another computer through a network, not shown. As non-volatile storage device 2080, a hard disk (HDD), a solid state drive (SSD) or the like may be used. Non-volatile storage device 2080 corresponds to storage unit 36.

By operation processes executed by CPU 2040 in accordance with a program, various functions of data processing unit 32 including, for example, functions of control unit 42, data collecting unit 46 and image processing unit 48 are realized.

A program or programs causing data processing unit 32 to execute the function of the present embodiment as described above may be stored on a CD-ROM 2200 or in a memory medium 2210 and inserted to disk drive 2030 or memory drive 2020 and may be further transferred to non-volatile storage device 2080. The program or programs will be loaded to RAM 2070 when executed.

Data processing unit 32 further includes a keyboard 2100 and a mouse 2110 as input devices, and a display 2120 as an output device. Keyboard 2100 and mouse 2110 correspond to input unit 40 and display 2120 corresponds to display unit 38.

The program or programs realizing the functions of data processing unit 32 as described above may not necessarily include an operating system (OS) for executing the functions of information processing apparatus such as computer main body 2010. The program or programs may only include those portions of instructions which can call appropriate functions (modules) in a controlled manner to attain a desired result. The manner how data processing unit 32 operates is well known and, therefore, detailed description will not be given here.

Further, the above-described program or programs may be executed by one computer or by a plurality of computers. In other words, both centralized processing and distributed processing are possible.

Alternatively, measurement may be done by one computer and calculation for obtaining feedback information based on the measurements may be done by another computer, and these computers may be connected by a network.

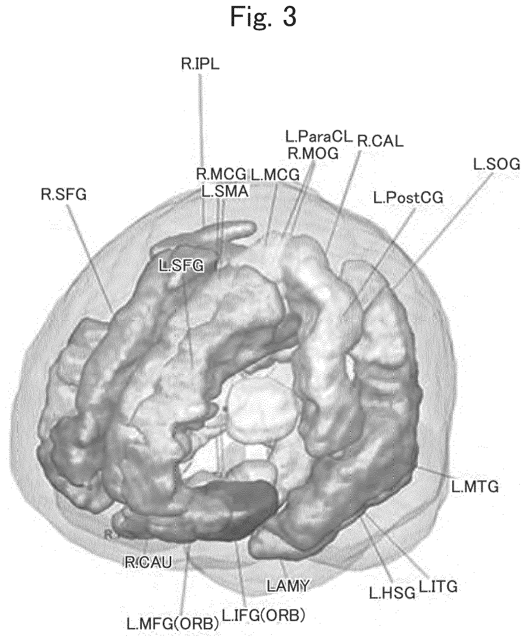

Fig. 3 shows regions of interest (ROIs) of a brain imaged by rs-fcMRI in accordance with an embodiment.

Here, 140 regions are used as anatomically divided regions of interest.

Though not specifically limited, Brodmann areas may be used as the anatomically divided regions of interest.

Such ROIs include, by way of example, the following:

Dorsomedial Prefrontal Cortex (DMPFC);
Dorsolateral Prefrontal Cortex (DLPFC)
Ventromedial Prefrontal Cortex (VMPFC);
Precuneus and Posterior Cingulate Cortex
Anterior Cingulate Cortex (ACC);
Cerebellar Vermis;
Left Thalamus;
Right Inferior Parietal Lobe;
Right Caudate Nucleus;
Right Middle Occipital Lobe; and
Right Middle Cingulate Cortex ....
It is noted, however, that the brain areas used may not be limited to those above.

For instance, the regions to be selected may be changed in accordance with the neurological/mental disorder to be studied or contents of intended brain function training.

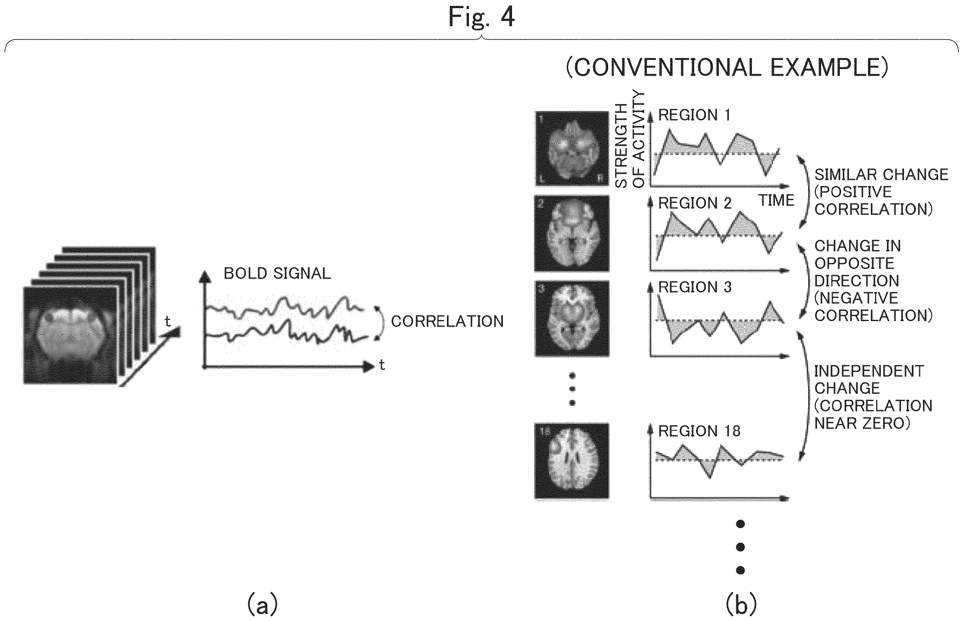

Fig. 4 shows a concept of correlation of changes over time in activities of brain areas in a resting state, or functional connectivity among brain areas in the resting state.

As shown in Fig. 4(a), a specific region of interest is subjected to time-sequential fMRI measurement and changes over time in BOLD signal is measured. Thus, changes over time in the degree of activity (level of activity) of the region of interest can be measured.

Then, by comparing changes over time in the degrees of activity of two regions of interest, it becomes possible to evaluate correlation of activities (functional connectivity of brain activities).

Specifically, as shown in Fig. 4(b), typically, there are supposed to be three types of correlation between two regions.

i) If activities of two regions change in a similar manner with time as can be seen between "region 1" and "region 2", we can say that these two have positive correlation. Here, it is expressed as "functional connectivity between brain regions is positive."

ii) If activities of two regions change in opposite directions with time as can be seen between "region 2" and "region 3", we can say that these two have negative correlation. Here, it is expressed as "functional connectivity between brain regions is negative."

iii) If activities of two regions change independently from each other with time as can be seen between "region 3" and "region 18", we can say that these two have near-zero correlation. Here, it is expressed as "functional connectivity between brain regions is non-existent."

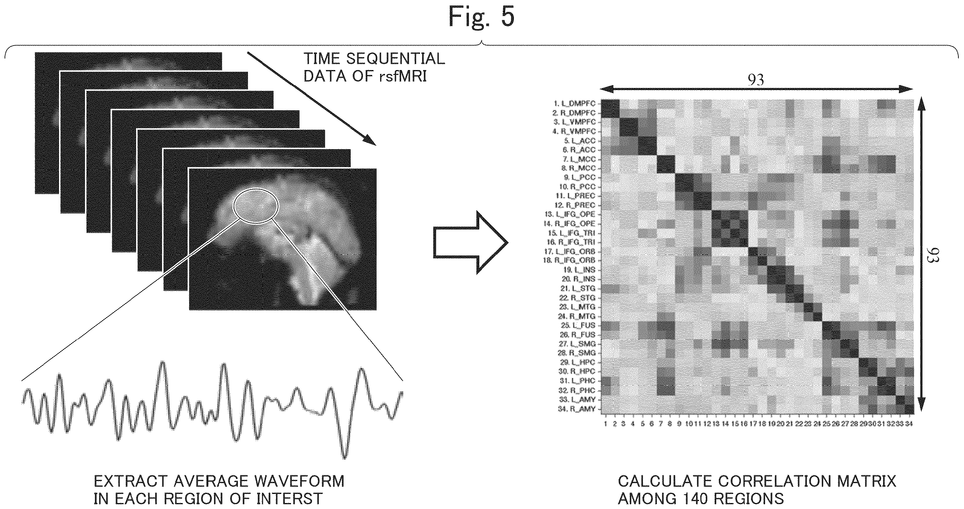

Fig. 5 shows a concept of a procedure for extracting a correlation matrix representing correlations of functional connections in the resting state, from regions of interest such as shown in Fig. 3.

Such a concept and the procedure for forming a discriminator are disclosed in Patent Literature 2: WO-A1-2014/178323 and Patent Literature 3: WO-A1-2017/090590 mentioned above and, therefore, only the outlines thereof will be described here.

As shown in Fig. 5, from fMRI data of n (n: a natural number) time points in the resting state measured on a real-time basis, an average "degree of activity" of each region of interest is calculated, and correlations among the brain regions (among the regions of interest) are calculated.

Here, 140 regions are picked up as regions of interest as noted above and, therefore, the number of independent non-diagonal elements in the correlation matrix will be, considering the symmetry,

$$(140 \times 140 - 140)/2 = 9730.$$

In Fig. 5, only the correlations of 34 $\times$ 34 are shown.

Though not specifically limited, calculation of such elements of a correlation matrix may be executed in the following manner.

From the data of brain activities in the resting state, for each subject, Functional Connectivity (FC) among different regions of interest (ROIs) are calculated. FC is a feature generally used in analyzing brain activity in the resting state, and it is defined by Pearson's correlation coefficient between different ROI time-sequential signals.

Calculation of correlation coefficient will be described later.

As for ROIs, in addition to 137 ROIs included in Brain Sulci Atlas (BAL), ROIs of cerebellums (left and right) and vermis of Automated Anatomical Labeling Atlas are used. Functional connections (FC) among these 140 ROIs are used as features.

Here, Brain Sulci Atlas (BAL) and Automated Anatomical Labeling Atlas are disclosed in the following references.

> Reference: Perrot et al., Med Image Anal, 15 (4), 2011
> Reference: Tzourio-Mazoyer et al, Neuroimage, 15 (1), 2002

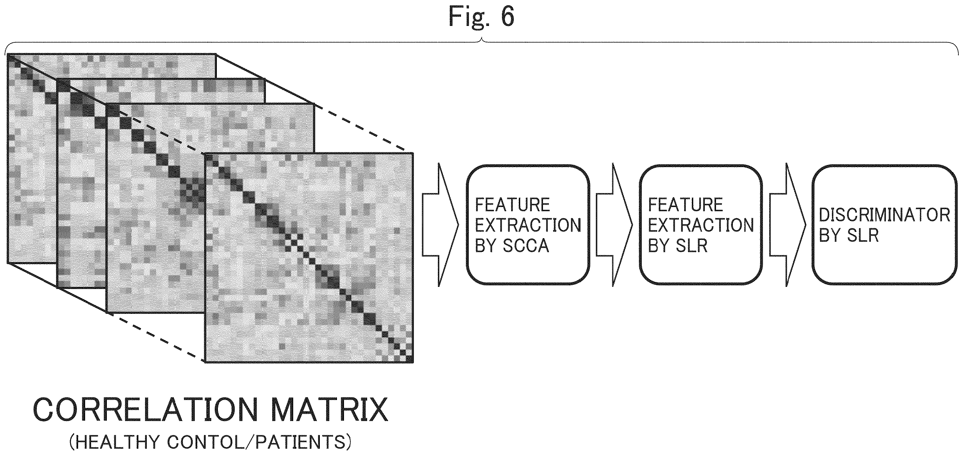

Fig. 6 shows a concept of a process for generating a discriminator serving as a biomarker, from the correlation matrix described with reference to Fig. 5.

Referring to Fig. 6, from data of resting-state functional connection MRI obtained by measuring a group of healthy subjects and a group of patients, data processing unit 32 derives a correlation matrix of degrees of activities among brain regions (regions of interest) in accordance with a procedure that will be described later.

Thereafter, by data processing unit 32, feature extraction is performed by regularized canonical correlation analysis on the correlation matrix and on the attributes of subjects including disease/healthy labels of the subjects. Here, "regularization" generally refers to a method of preventing over-learning by adding a regularization term weighted by hyper parameters to an error function, in machine learning and statistics, and thereby restricting complexity/degrees of freedom of a model. If the result of regularized canonical correlation analysis results in sparse explanatory variables, this process will be specifically referred to as a sparse canonical correlation analysis (SCCA). In the following, an example employing sparse canonical correlation analysis will be described.

In the above-described sparse canonical correlation analysis, when hyper parameter values are adjusted to allow the presence of a canonical variable that connects only to a "diagnosis label" as will be described later, a functional connection FC that connects to the corresponding canonical variable is extracted. When the hyper parameters are varied in a prescribed range, functional connections FC may be extracted within a range in which the canonical variables satisfying such conditions exist. A sum-set of such functional connections will be referred to as a "first sum-set."

Further, using the "first sum-set" obtained as a result of sparse regularized canonical correlation analysis, data processing unit 32 performs Leave-One-Out Cross Validation: LOOCV) while performing discrimination analysis by sparse logistic regression at each step of cross validation, whereby a sum-set of functional connections FC are extracted as explanatory variables over all the cross validations, which set will be referred to as a "second sum-set."

Finally, the data of all subjects are subjected to discrimination analysis by sparse logistic regression of "diagnosis label" as the object variable, using the "second sum-set" as explanatory variables, and thus, a discriminator is generated.

As will be described later, the data of the healthy group and the patient group are not limited to those measured by the MRI apparatus 10 itself. Data measured by a different MRI apparatus may also be integrated, to generate the discriminator. More generally, data processing unit 32 may not necessarily be a computer for executing control of the MRI apparatus, and it may be a computer specialized in generating the discriminator by receiving measurement data from a plurality of MRI apparatuses and performing the discriminating process by using the generated discriminator.

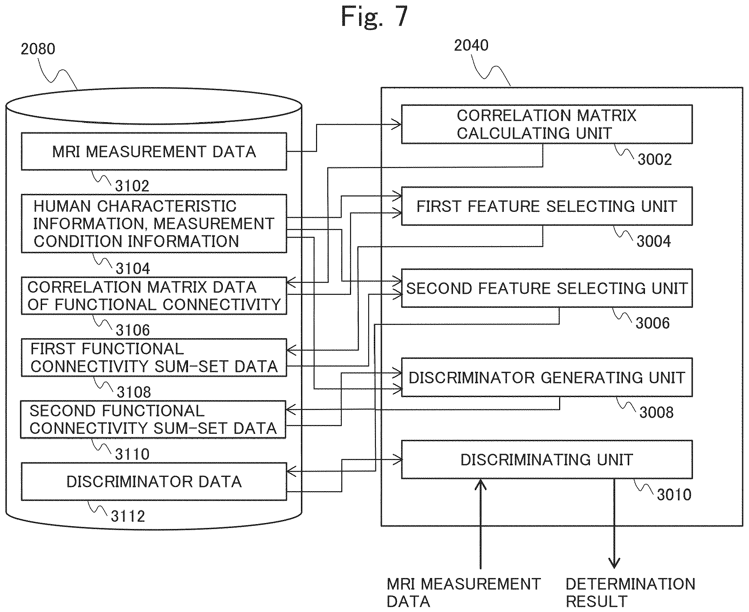

Fig. 7 is a functional block diagram for a discriminator generating process such as shown in Fig. 6 and a discriminating process by the generated discriminator.

First, a non-volatile storage device 2080 stores MRI measurement data 3102, which is information of signals measured time-sequentially in advance by the MRI device as signals representing brain activities in a prescribed plurality of regions in the brain of each of the subjects; and pieces of human attribute information 3104 associated with each of the subjects whose MRI measurement data are measured.

Here, "human attribute information" includes pieces of "human characteristic information" for specifying individual subject and "measurement condition information" for specifying measurement conditions for each subject.

"Human characteristic information" means information such as diagnosis label, age, sex, medication profile and the like of each subject.

"Measurement condition information" includes information of measurement site where measurement of the subject took place (including information specifying the measurement facility and/or device used for measurement) and conditions such as whether the eyes were open/closed during measurement, as well as conditions for measurement such as magnetic field strength for measurement.

Processor 2040 performs the process of generating a discriminator for the diagnosis label based on the MRI measurement data 3102 and the corresponding human attribute information 3104.

A correlation matrix calculating unit 3002 calculates, for each subject, the correlation matrix of functional connection of brain activities among a prescribed plurality of regions from the MRI measurement data 3102. The calculated data of the correlation matrix of functional connection is stored, subject by subject, as correlation matrix data 3106 of functional connection, in non-volatile storage device 2080.

A first feature selecting unit 3004 successively selects, from K (K: a natural number not smaller than 2) different subsets extracted from a plurality of subjects, one subset and performs a sparse canonical correlation analysis on pieces of attribute information and on elements of the correlation matrix of (K-1) subsets except for the selected one subset, and thereby extracts elements of a correlation matrix that connects to a canonical variable corresponding only to a specific piece of attribute information among the pieces of human attribute information, for example, the diagnosis label. Further, the first feature selecting unit 3004 obtains, for the successively selected subsets, the first sum-set as the sum-set of extracted elements of correlation matrix, and stores as first functional connection sum-set data 3108 in non-volatile storage device 2080. Here, the "first functional connection sum-set data" may be indices for specifying elements corresponding to the first sum-set, among the functional connection correlation matrix data 3106.

Subjects other than the above-described K subsets of the plurality of subjects are used as a test set, which test set is divided into N different groups; the second feature selecting unit 3006 calculates by sparse logistic regression a test discriminator for estimating the above-described specific attribute information (for example, the diagnosis label) based on the first sum-set, on a set of subjects except for one group selected from the N groups of the plurality of subjects; and extracts, along with sparsing, elements of the correlation matrix as the explanatory variables of the test discriminator. Further, the second feature selecting unit 3006 repeats feature extraction while successively selecting one group from N groups to obtain the second sum-set as the sum-set of elements of the correlation matrix extracted as the explanatory variables of test discriminator, and stores it as the second functional connection sum-set data 3110 in non-volatile storage device 2080. Here again, the "second functional connection sum-set data" may be indices for specifying elements corresponding to the second sum-set among the functional connection correlation matrix data 3106.

It is noted that the number of elements in each of the N groups may be one.

A discriminator generating unit 3008 calculates by the sparse logistic regression a discriminator for estimating a specific attribute information (for example, the diagnosis label) using the second sum-set as explanatory variables. Discriminator generating unit 3008 stores information for specifying the generated discriminator as discriminator data 3112 in non-volatile storage device 2080.

A discriminating unit 3010 performs a discriminating process on input data based on the discriminator specified by discriminator data 3112.

In the description above, discriminator generating unit 3008 has been described as generating a discriminator using the second sum-set as explanatory variables. Alternatively, discriminator generating unit 3008 may generate a discriminator by directly using the first sum-set as the explanatory variables. From the viewpoint of reducing the number of dimensions and of improving generalization, however, it is desirable to use the second sum-set as the explanatory variables.

In generating a discriminator, it is possible to use regularized logistic regression, which is logistic regression with regularization (for example, L1 regularization or L2 regularization). More specifically, the above-described "sparse logistic regression" may be used. Further, for generating a discriminator, a support vector machine or LDA (linear discriminant analysis) may be used. In the following, an example using sparse logistic regression will be described.

As will be described later, in parallel with the feature selecting process by the second feature selecting unit 3006, using the test discriminator calculated by the second feature selecting unit 3006, while successively selecting one group to be excluded from the N groups, the excluded group may be used as a test sample and the result of discrimination may be calculated, and whereby cross-validation may be effected.

By reducing the dimension of explanatory variables by the nested feature selecting procedure, it becomes possible to realize time-efficient reduction of the number of dimensions, while using the data of almost all subjects, in the process performed by the second feature selecting unit 3006.

Further, by making the test set in the process of second feature selecting unit 3006 independent from the data set used for reducing the number of dimensions, it becomes possible to avoid excessively optimistic results.

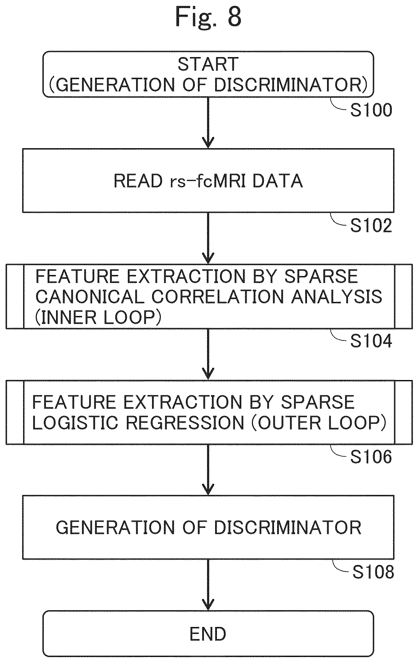

Fig. 8 is a flowchart representing a process data processing unit 32 executes for generating the discriminator serving as the biomarker.

In the following, the process described with reference to Fig. 6 will be discussed in greater detail with reference to Fig. 8.

The biggest problem posed when a biomarker is to be generated based on the discriminant label (diagnosis label) of a disease of a subject and connections of brain areas derived from the fMRI data in the resting state is that the number of data dimensions is overwhelmingly larger than the number of data. Therefore, if training of a discriminator for predicting the diagnosis label (here, the label indicating whether the subject has the disease or healthy will be referred to as the "diagnosis label") is done using a data set without regularization, the discriminator will be over-fitted, and the prediction performance for unknown data significantly decreases.

Generally, in machine learning, a process to enable explanation of observed data with a smaller number of explanatory variables is referred to as a "feature selection (or feature extraction)." In the present embodiment, "extraction of contraction expression" refers to a feature selection (feature extraction) to enable formation of the discriminator with smaller number of correlation values in the machine learning of the discriminator for predicting the diagnosis label of a disease to be studied from among "a plurality of correlation values (a plurality of connections) of the degrees of activities among the brain regions (regions of interest)," that is, to select correlation values of higher importance as the explanatory variables.

In the present embodiment, as the method of feature extraction, regularization is adopted. In this manner, canonical correlation analysis is performed with regularization and sparsing, so as to leave explanatory variables of higher importance. This process is referred to as "sparse canonical correlation analysis." More specifically, as the method of regularization that also realizes sparsing, we can use a method of imposing a penalty to the magnitudes of absolute values of parameters for canonical correlation analysis referred to as "L1 regularization" as will be described in the following.

Specifically, referring to Fig. 8, when the process for generating the discriminator starts (S100), data processing unit 32 reads MRI measurement data of each subject from storage unit 36 (S102), and performs the first feature extraction process by the sparse canonical correlation analysis (SCCA) (S104).

In the following, the process at step S104 will be referred to as an "inner loop feature selection."

Here, in order to facilitate understanding of the inner loop feature selection process at step S104, the "sparse canonical correlation analysis" and the "inner loop feature selection" will be described.

(Sparse Canonical Correlation Analysis)

In the following, L1 regularization canonical correlation analysis will be described as the sparse canonical correlation analysis. As to the L1 regularization canonical correlation analysis, see, for example, the reference below.

Reference: Witten DM, Tibshirani R, and T Hastie. A penalized matrix decomposition, with applications to sparse principal components and canonical correlation analysis. Biostatistics, Vol. 10, No. 3, pp. 515-534, 2009.

First, in the general Canonical Correlation Analysis (CCA), when a variable pair $x_1$ and $x_2$ is to be considered, the variables $x_1$ and $x_2$ are each standardized to have an average zero and standard deviation one.

Generally speaking, by using the canonical correlation analysis (CCA), it becomes possible to identify the latent relation between paired observables.

Specifically, in the canonical correlation analysis, a projection vector is found such that projected pair of variables (canonical variables) have the highest correlation.

In contrast, when the L1 regularization is introduced to the canonical correlation analysis, that is, when the sparse CCA with L1 norm regularization or L1-SCCA is used, the process will be to solve the following optimization problem.

Consider the following sets of variables.

[Expression 1]

Where there are N sets of observed values of variables $x \in \mathbf{R}^{p1}$ and $x \in \mathbf{R}^{p2}$, $\mathbf{X}_1 = \left[ x_1^1, x_1^2, \cdots, x_1^N \right]^T$

$$\mathbf{X}_1 = \left[ x_2^1, x_2^2, \cdots, x_2^N \right]^T$$

represents a N×p$_1$ matrix comprised of a first set of variables, and represents a N×p$_2$ matrix comprised of a second set of variables. Here again, it is assumed that the columns forming matrices X$_1$ and X$_2$ are each normalized to have an average zero and variance one.

Then, L1-SCCA can be formulated as Equation (1) below.

[Expression 2]

$$\max_{\mathbf{w}_1, \mathbf{w}_2} \mathbf{w}_1^T \mathbf{X}_1^T \mathbf{X}_2 \mathbf{w}_2 \quad subject\ to\ \left\|\mathbf{w}_1\right\|_1^2 \le \lambda_1, \left\|\mathbf{w}_2\right\|_1^2 \le \lambda_2, \left\|\mathbf{w}_1\right\|_2^2 \le 1, \left\|\mathbf{w}_2\right\|_2^2 \le 1 \quad (1)$$

Here, hyper parameters $\lambda_1$ and $\lambda_2$ respectively represent the degrees of sparseness (referred to as "sparse projection vectors" because the variables corresponding to $w_1$ and $w_2$ are made sparse) of weight parameters $w_1$ and $w_2$.

Specifically, in the present embodiment, in order to identify the latent relations of the functional connections FC and the human attribute information, two data matrixes corresponding to the above-described variables are formed.

In the following, the subjects include a group of melancholic depression patients (whose diagnosis label is indicated as "MDD (Melancholic Depression Disorder)" and a group of healthy people (whose diagnosis label is indicated as "HC (Healthy Control)". The column of the first data matrix $X_1$ represents the human attribute information (human characteristic information and measurement condition information) of one subject, and the characteristic information and measurement condition information include the following:

i) diagnosis label (MDD or HC)
ii) site information (at which site the brain activities of the subject were measured, site A, B or C)
iii) age
iv) sex
v) conditions of imaging (eyes open or closed)
vi) state of first medication (antipsychotic medicine)
vii) state of second medication (antidepressant)
viii) state of third medication (tranquillizer)

Specifically, the number of columns of the human attribute information data matrix $X_1$ is 10, that is, pi = 10.

The first column includes 1 (= MDD) or 0 (=HC).

The following three columns indicate facilities (sites) where the brain activities were measured and, in this example, these columns include any of [100] (site A), [010] (site B) and [001] (site C), corresponding to measurements that took place at three different sites.

The fifth column includes a value indicating the age of the subject; the sixth column represents information of the sex of the subject, and it includes either the value 1 (male) or 0 (female); the seventh column indicates, among the conditions of measurement, whether the subject's eyes were open or closed during the measurement, and it includes the value 1 (eyes open) or 0 (eyes closed). The last three columns includes status information related to medication profile of three medicines and each column includes a value 1 (medicine administered) or 0 (medicine not administered), respectively.

The human characteristic information and the measurement condition information are not limited to those described above. For example, other characteristics such as information of the medical profile related to other medicines of the subjects, or other conditions of measurement such as the magnitude of the magnetic field applied by the MRI apparatus, may be included.

The second data matrix $X_2$ represents, in the form of a row vector, elements at the off-diagonal lower triangle of the correlation matrix representing the correlation of the functional connections (FC) of the subject.

Then, L1-SCCA is applied to the pair of matrices $X_1$ and $X_2$, and thereby, sparse projection vectors $w_1$ and $w_2$ are derived.

Then, as described above, the following conditions are set to be satisfied: specifically, when the hyper parameters $\lambda_1$ and $\lambda_2$ are set to prescribed values, the first data matrix $X_1$ will be projected, by the sparse projection vector $w_1$, through sparsing, to a canonical variable representing only a specific piece of human attribute information: "diagnosis label" in this case. Here, in the corresponding second data matrix $X_2$, in order to specify an index (element) for identifying the element of the correlation matrix of that functional connection which relates only to the diagnosis label, the sparse projection vector $w_2$ is used.

Specifically, in the inner loop feature selection, hyper parameters $\lambda_1$ and $\lambda_2$ of L1-SCCA are changed independently between 0.1 and 0.9 at a step of, for example, 0.1.

The range and the magnitude of the steps of changing the hyper parameters $\lambda_1$ and $\lambda_2$, however, are not limited to such examples.

For the process of L1-SCCA, the range of hyper parameters $\lambda_1$ and $\lambda_2$ in which such a canonical variable that is connected only to the "diagnosis" label exists, should be found.

Here, projection of elements of the original correlation matrix to a subspace defined by non-zero elements of derived sparse projection vector $w_2$ is represented as follows.

Here, a variable $i_k$ is defined to indicate an index of a k-th non-zero element of the sparse projection vector $w_2$. Here, $1 \le k \le m$, and m represents the number of non-zero elements.

Then, consider a projection matrix E to a subspace as below.

[Expression 3]

$$E = [\mathbf{e}_{i_1}, \quad \mathbf{e}_{i_2}, \quad \cdots \quad \mathbf{e}_{i_m}]^{\mathrm{T}}$$

Here, $\mathbf{e}_{i_k} \in \mathbf{R}^{p_2}$ is a standard basis vector including a "1" in the $i_k$-th element and zeroes in other elements.

Finally, by projecting the element $x_2$ of the original correlation matrix in the following manner, a vector to the subspace $z \in R^m$ is derived.

[Expression 4]

$$z = Ex_2$$

As a result, it is possible to select a specific number of features (elements of the correlation matrix) that relate to the diagnosis label (MMD/HC).

By selecting elements of the correlation matrix that correspond to the canonical variables related only to the diagnosis label, elements of the correlation matrix that are essential to classification can be selected.

In other words, as the condition of constraint for L1 regularization is added, of the elements of parameters $w_1$ and $w_2$, elements having low importance will have the value 0 and thus, the features (explanatory variables) will be made sparse.

Thereafter, data processing unit 32 executes discriminant analysis using the Sparse Logistic Regression (SLR) based on the results of sparse canonical correlation analysis (S106).

Thereafter, data processing unit 32 executes the second feature extraction process (S106) using the sparse logistic regression based on the results of the inner loop feature selection.

In the following, the process at step S106 will be referred to as an "outer loop feature selection."

Here, in order to facilitate understanding of the outer loop feature selection process at step S106, the "sparse logistic regression" and the "outer loop feature selection" will be described, respectively, in the following.

(Sparse Logistic Regression)

The sparse logistic regression refers to a method of a logistic regression analysis expanded to a Bayes' estimation scheme, in which dimensional compression of a feature vector is performed concurrently with weight estimation for discrimination. This is useful when the number of the dimensions of the feature vector is very large and includes many unnecessary feature elements. For unnecessary feature elements, weight parameters in linear discrimination analysis will be set to zero (that is, features are selected), so that only a limited number of feature elements related to the discrimination are extracted (sparseness).

In the sparse logistic regression, the probability p of the obtained feature data belonging to a class is calculated class by class, and the feature data is classified to the class corresponding to the highest output value. The value p is output by a logistic regression equation. Weight estimation is done by ARD (Automatic Relevance Determination), and feature elements with feeble contribution to class determination are removed from the calculation as its weight comes closer to zero.

Specifically, using the first sum-set of features extracted by the L1 regularization CCA as described above as inputs, the first discriminator based on the hierarchical Bayes' estimation as will be described in the following estimates the diagnosis label.

Here, in order to predict the probability of the diagnosis label indicating a disease (here, diagnosis of melancholic depression MDD) from the feature input z (selected FC) extracted by Equation (2) above, logistic regression is utilized as a discriminator.

[Expression 5]

$$p(y = 1 \mid \boldsymbol{\theta}) = \frac{1}{1 + \exp(-\boldsymbol{\theta}^T \hat{\mathbf{z}})} \quad (3)$$

Here, y represents diagnosis class/label. Specifically, y = 1 represents the MDD class and y = 0 represents the HC class.

Further, the following z hat (a character with "∧" above will be referred o as "hat") represents a feature vector with an extended input.

[Expression 6]

$$\hat{\mathbf{z}} = \left[\mathbf{z}^T, \quad 1\right]^T \in \mathbf{R}^{m+1}$$

Here, the feature vector z is extracted in accordance with Equation (2) from the connection correlation matrix of MRI samples of one subject in a resting state.

Use of extended input "1" is a standard approach for introducing a certain (bias) input to the first discriminator.

θ shown in the following is a parameter vector of a logistic function.

[Expression 7]

$$\mathbf{\theta} \in \mathbf{R}^{m+1}$$

Here, the distribution of parameters θ is set to a normal distribution given as

[Expression 8]

$$p(\mathbf{\theta} \,|\, \alpha) = N(\mathbf{\theta} \,|\, 0, diag(\alpha))$$

Further, by setting the distribution of hyper parameters α of distribution of parameter w as follows, distribution of each parameter is estimated by hierarchical Bayes' estimation.

[Expression 9]

$$p(\alpha) = \prod_{j} \Gamma(\alpha_i \,|\, a^0, b^0)$$

Here, $a^0$ and $b^0$ are parameters determining gamma distribution of hyper parameters. α is a parameter vector representing variance of normal distribution of vector θ, and the i-th element of vector α is represented by $\alpha_1$.

As to the sparse logistic regression, see, for example, the following reference.

Reference: Okito Yamashita, Masa aki Sato, Taku Yoshioka, Frank Tong, and Yukiyasu Kamitani. "Sparse Estimation automatically selects voxels relevant for the decoding of fMRI activity patterns." NeuroImage, Vol. 42, No. 4, pp. 1414-1429, 2008.

Thereafter, data processing unit 32 executes the process of generating the discriminator (S108) by the sparse logistic regression, based on the results of the outer loop feature selection (second sum-set).

(Imaging Conditions of Resting State fMRI as Biomarker and Extraction of Correlation Matrix)

Resting state fMRI imaging device is not limited to the one described above provided that the functions shown in Figs. 1 and 2 can be realized.

Conditions of imaging are not limited, either, as long as fMRI images can be obtained. Examples are as follows: magnetic field: about 3.0T, field of view: about 192 mm to about 256 mm, matrix: about 64 × 64, number of slices: about 30 to about 40, number of volumes: about 112 to about 244, slice thickness: about 3.0 mm to about 4.0 mm, slice gap: about 0 mm to about 0.8 mm, TR: about 2,000 ms to about 2,700 ms, TE: about 25 ms to about 31 ms, total imaging time: about 5 min. to about 10 min, Flip angle: about 75 deg to about 90 deg, slice acquisition order: Ascending (interleaved). Imaging should preferably be done in dim lighting. Further, it is preferred that a subject stays awake and blank headed during imaging. Further, it is preferred that the subject keeps looking at a cross-mark at the center of a monitor screen.

The obtained fMRI images can be processed by the method described in Yahata et al., Non-Patent Literature 10.

From pre-processed image data, the functional connectivity (FC) (that is, strength of connection) among a prescribed number of regions of interest (ROIs) of the brain is calculated. Functional connectivity is a feature (elements of correlation matrix) generally used in analyzing brain activity in the resting state, and it is defined by Pearson's correlation coefficient between different ROI time-sequential signals.

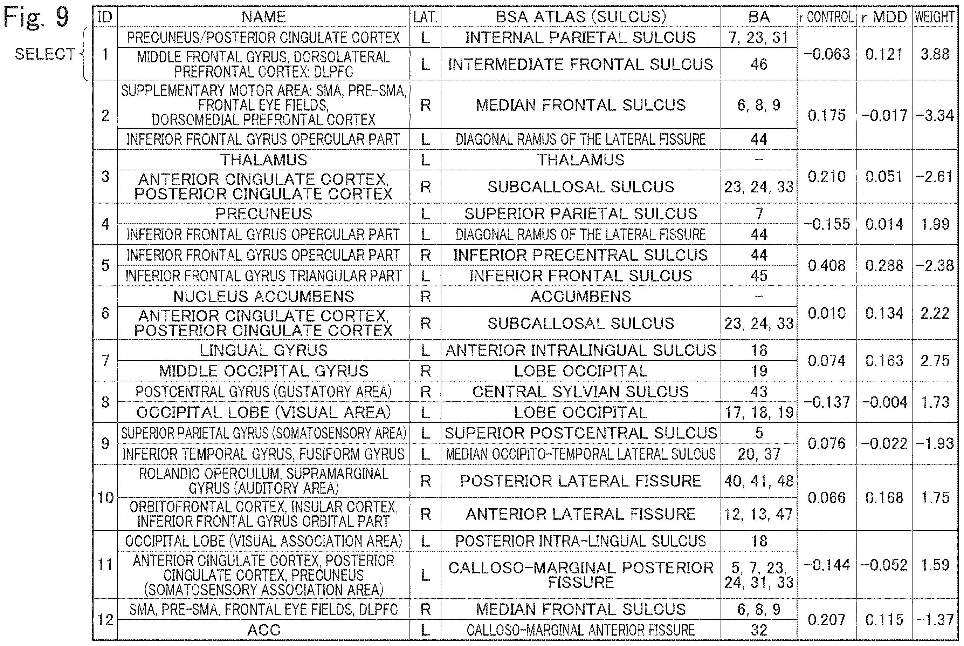

Fig. 9 shows functional connectivities extracted for discriminating a state indicating symptoms of depression disclosed in Non-Patent Literature 1 mentioned above.

As shown in Fig. 9, in order to discriminate a state indicating symptoms of depression, functional connectivity (strength of connection) is calculated between every one of, or some of ROIs denoted by functional connectivity identification number (ID in Fig. 9) 1 to 12.

In Fig. 9, "L" and "R" of Lat. distinguish Left and Right brains. BSA (Brainvisa Sulci Atlas) represents anatomical areas with reference to the cerebral sulcus and BA represents the number of Brodmann areas corresponding to the BSA area. Weight indicates relation weighted sum (also simply referred to as "weighted sum").

"Relation weighted sum" means the value of "$\theta^t \cdot z$ hat" in the exp function of the denominator of Equation (3) above. Therefore, "weight" refers to each element of $\theta$.

Though not specifically limited, calculation of such elements of a correlation matrix may be executed in the following manner.

First, time sequence of mean signals of all voxels included in each ROI is extracted. Thereafter, band-pass filtering (0.008 to 0.1 Hz) is conducted for noise cancellation of signal values, followed by regression using nine explanatory variables (mean signals of whole brain, white matter, brain spinal fluid and six motion correction parameters). Residual error sequence after regression is regarded as a time-sequential signal values related to functional connections, and Pearson's correlation coefficient is calculated for elements of respective correlation matrixes of regions of interest of each pair of ROIs. The correlation coefficient described above is the value representing connection strength of functional connectivity, and for each pair, corresponding connection strength is calculated.

By inputting the correlation coefficient of each functional connectivity as described above as input data to a discriminator, which will be described later, an index for discriminating a depression disorder label is calculated for the functional connectivity, based on the above-described correlation coefficient and weight (degree of contribution) calculated in advance for each functional connectivity for executing the discriminating process by the discriminator. Alternatively, a relation weighted sum of a plurality of functional connectivities is calculated as an index for discriminating a depression disorder label, based on the above-described correlation coefficient and the above-described coefficient. Here, "relation weighted sum" refers to a sum of the plurality of functional connectivities respectively multiplied by corresponding weights.

Therefore, the index mentioned above is not the mere data of measurement of brain activities of the subject but an artificially calculated value in consideration of the weight of each functional connectivity. The index is used for discriminating a label of depression symptoms, for discriminating a level of depression symptoms, for discriminating therapeutic effect or for stratifying patients of depressive disorder.

Of the twelve pairs of functional connectivities described above, the one having the highest contribution to the depression symptoms is the functional connectivity denoted by functional connectivity Identification Number 1, and the one having the second highest connectivity is denoted by functional connectivity Identification Number 2. Therefore, in the embodiments described in the following, at least one of, or both of functional connectivity IDs 1 and 2 may be selected and used.

The twelve pairs of functional connectivities described above are particularly suitable for discriminating a group of melancholic MDD patients from healthy control.

(Generation of Discriminator and Discriminating Device)

The discriminator is generated by a discriminator generating process from signals time-sequentially measured beforehand by a brain activity detecting device that detects signals indicating brain activities in a plurality of prescribed brain regions of each of a plurality of participants including healthy people and patients of depression disorder in the resting state. Here, the patient of depression disorder means a participant who is diagnosed beforehand by a doctor/physician to have depression disorder and is related to a "disorder label" of depression. The discriminator mentioned above is generated such that the disorder label of depression symptoms is discriminated based on the weight or weights of functional connectivities selected by a feature selection as being related to the disorder label of depression symptoms, through machine learning, from the plurality of functional connectivities between the plurality of prescribed regions.

Specifically, from the functional connectivities of a preset plurality of ROIs, some functional connectivities are extracted by the sparse canonical correlation analysis as differentially related to the disorder label of depression symptoms, and from the extracted functional connectivities, those used for discriminating the disorder label of the depression disorder are selected by a feature selection using the sparse logistic regression. Then, from the functional connectivities selected in this manner, the above-described relation weighted sum is calculated.

(Connectivity Neurofeedback)

In the following, a brain activity training device that provides feedback to brain activities of a trainee to help attain the state of correlation (connectivity) of healthy people (neurofeedback training or simply, training) will be described.

More generally speaking, the brain activity training device as described above may be used not only for the training to make the functional connectivity state of a trainee closer to the functional connectivity state of brain activities of healthy people in the context of brain activities among a patients group and healthy control but also for a training to make present functional connectivity state of a trainee closer to a functional connectivity state of aimed brain activities. The functional connectivity state of brain activities of healthy people or the functional connectivity state of aimed brain activities (time correlation of activity signals of brain regions corresponding to functional connectivities) will be referred to as "target pattern" as will be described later.

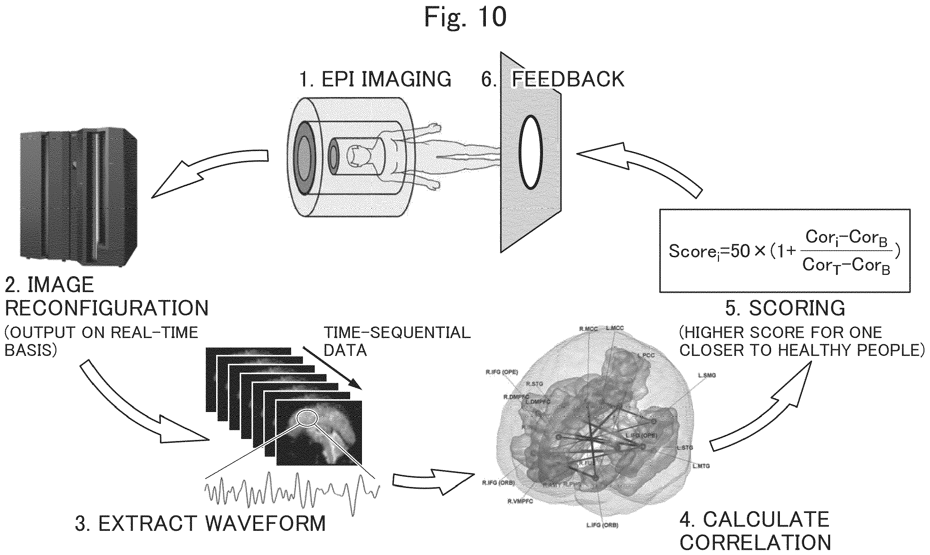

Fig. 10 shows a concept of the process executed by the brain activity training apparatus.

The brain activity training device may have the same hardware configuration as that of MRI apparatus 10 shown in Fig. 1.

In the method of driving the brain activity training device described in the following, it is assumed that real-time fMRI of multiband imaging is used as the brain activity detecting device for time-sequentially measuring signals indicating brain activities by functional brain imaging.

Referring to Fig. 10, first, by the MRI apparatus 10, brain activities of a trainee are time-sequentially measured for a prescribed time period. To realize fMRI, EPI (Echo-Planar Imaging) is executed as multiband imaging.

Thereafter, by data processing unit 102, the obtained images are re-configured on real-time basis.

Here, functional connectivities of regions of interest denoted by functional connectivity Identification Numbers (ID in Fig. 9) 1 to 12 shown in Fig. 9 are selected for discriminating the label of "depression symptoms."

Specifically, in the neurofeedback training of a trainee, at least one specific functional connectivity among the above-described twelve functional connectivities is further selected/extracted as "training target functional connectivity."

Though not specifically limited, as the "training target functional connectivity," of the functional connectivities shown in Fig. 9, "functional connectivity between left Dorsolateral Prefrontal Cortex (DLPFC) and left Precuneus and left posterior cingulated cortex (PCC)" (connectivity of ID = 1 in Fig. 9), which has the highest degree of contribution to the discrimination of the depression disorder label, is selected as the object. Here, the "degree of contribution" means the "absolute value of the weights in the weighted sum."

It is noted that as the "training target functional connectivity," only the connectivity of ID = 1 may be selected or, in addition to the connectivity of ID = 1, other connectivity may be additionally selected. Here, as the functional connectivity selected in addition to the connectivity of ID = 1, the connectivity having the second highest connectivity, that is, ID = 2, may be selected. The number of functional connectivities to be selected in addition to the connectivity of ID = 1 may be one or more.

In the following, description will be made assuming that only the ID = 1 connectivity is selected as the "training target functional connectivity."

Further, data processing unit 102 extracts waveforms for a prescribed time period for the regions in accordance with the regions of interest corresponding to the "training target functional connectivity," and extracts time correlation of functional connectivity in the prescribed time period.

Then, data processing unit 102 calculates a score to be fedback, as will be described later, in accordance with the calculated correlation value, transfers this to a diagram and presents it to the subject.

Here, the score is set to be higher if the state is closer to that of the healthy people.

Data processing unit 102 displays the diagram corresponding to the calculated score SC on a display 6, as a feedback to trainee 2.

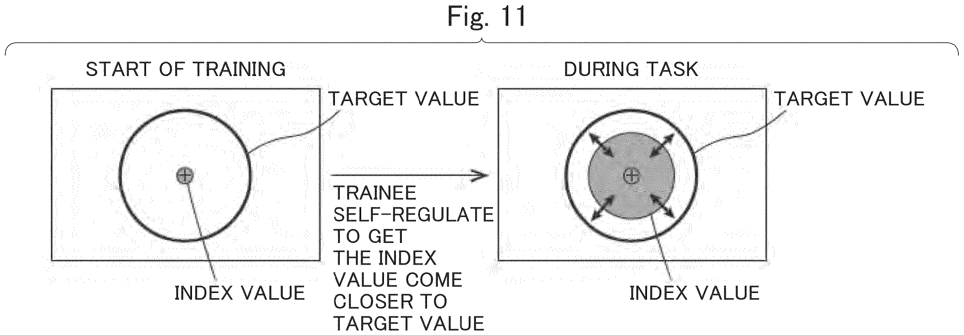

Fig. 11 shows an example of a diagram presenting information to be fed back to a subj ect.

The information to be fedback may be the score value itself. Alternatively, as is shown in Fig. 11, it may be a diagram of which size changes in accordance with the magnitude of the value. Any other diagram or the like may be used provided that the trainee can recognize the magnitude of the score.

In Fig. 11, the magnitude of score can be recognized by the subject as the radius of presented circle.

Thereafter, the process from EPI imaging to score feedback is repeated for a prescribed time period on real-time basis.

The method of score feedback is not limited to the above-described method in which a two-dimensional size of a specific diagram is given as a feedback. By way of example, other information such as sound may be used for feedback. When sound is used, the sound pitch may be made higher as the score increases, to let the subject recognize the score magnitude.

It is noted, however, that when the feedback is given utilizing the size of a specific diagram and a characteristic length of the specific diagram (for example, radius in the case of a circle and length of a side in the case of a square) is adjusted to be in proportion to the score, the area of the specific diagram comes to be in proportion to the square of the score. Therefore, the fact that the score is coming closer to the healthy state can be presented in an increasingly emphasized manner to the subject.

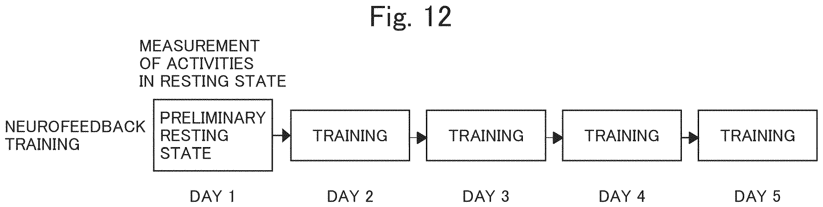

Fig. 12 shows an example of a neurofeedback training sequence.

As shown in Fig. 12, first, on day 1, brain activities of a trainee (hereinafter referred to as a "subject") before starting the neurofeedback training are measured.

Here, on day 1, the feedback value (score) is not presented to the subject, while the subject is instructed to do "self-regulation" only, in accordance with the same schedule as that for the feedback training from day 2 and onwards. For example, the training from day 2 involves presentation of feedback score as shown in Fig. 11, whereas on day 1, such circular diagram is not shown to the subject.

Here, regarding what we call "self-regulation of day 1," brain activities related to self-regulation for a number of times (for example, 20 to 60 times of trial) are measured, after instructing the subject to imagine calculating task or verbal fluency task, for example, as tasks to self-regulate. On the first day, it is detected to what degree of functional connectivity can be derived by the subject when he/she self-regulates about such a task, under the condition that no feedback information is presented. Then, as will be described later, mean value of the state at which the subject can derive the functional connectivity under the same condition (baseline state) is adjusted to be the feedback score of 50 points.

It is noted, however, that as the "baseline state," purely the brain activities in the resting state may be measured, without presenting any feedback information.

Thereafter, data processing unit 102 calculates values of object functional connectivities during self-regulation (time correlation values) of day 1 for the number of trials, and calculates a mean value of time correlation values (hereinafter referred to as baseline correlation value $Cor_B$).

Though not specifically limited, the trials for calculating the mean value are preferably done for the same period as "trials" during the neurofeedback training as will be described later and the number of trials is preferably the same as the "total number of trials."

In accordance with the baseline correlation value calculated in this manner, a target brain function connectivity value is set in accordance with a method that will be described in the following and, though not limited thereto, the feedback score is set such that it is 50 when the time correlation value of object functional connectivity during the feedback training is the same as the mean value mentioned above and it is 100 when it is the same as the target functional connectivity value.

By these operations, from day 2 and thereafter, the values of object functional connectivity calculated during the neurofeedback training are converted to neurofeedback scores.

On the other hand, images of brain activities in the resting state are taken every day during the neurofeedback training, in order to measure the effect of neurofeedback training.

After the above-described preparation of day 1, the neurofeedback training starts on day 2.

Thereafter, the neurofeedback training is continued for days 3 to 5.

The number of days for the training may be shorter or longer than described above.

(Calculation of Target Brain Function Connectivity Value ("Target Correlation Value $Cor_T$"))

Data processing unit 102 performs Fisher z-transformation on the baseline correlation value $Cor_B$ calculated in the above-described manner. Thus, it is now possible to handle z-transformed correlation value $zCor_B$ as a value that follows normal distribution.

Specifically, when the sample size n is sufficiently large, it is possible to consider that the correlation value $zCor_B$ approximately follows the normal distribution of mean $zCor_B$ and variance SD = $1/(n-3)$. By reverse transformation to the domain of the original correlation efficient, an interval estimation of the correlation coefficient can be obtained.

Here, let us consider an example in which a change of the correlation value after z-transformation (hereinafter referred to as "z correlation value") $zCor_B$ in a negative direction approximates the state of healthy people.

As described above, the baseline correlation value $Cor_B$ is calculated from the measurements data of brain activities of the subject on day 1. By Fisher z-transformation of thus calculated baseline correlation value $Cor_B$, it becomes possible to treat the time correlation value after z-transformation as normal distribution.

Assuming that the time correlation value after z-transformation follows normal distribution, the baseline correlation value after z-transformation and the value "baseline correlation value after z-transformation -1 $\times$ (variance)" (hereinafter denoted as "mean value - 1SD") are calculated.

Thereafter, by reverse z-transformation, "mean value - 1SD" is reverse-transformed to time correlation value, and the resulting reverse z-transformed value is the target brain function connectivity value (hereinafter denoted as "target correlation value Corr").

(Details of Neurofeedback Process)

In the following, details of executing neurofeedback training will be described.

(Multiband Imaging)

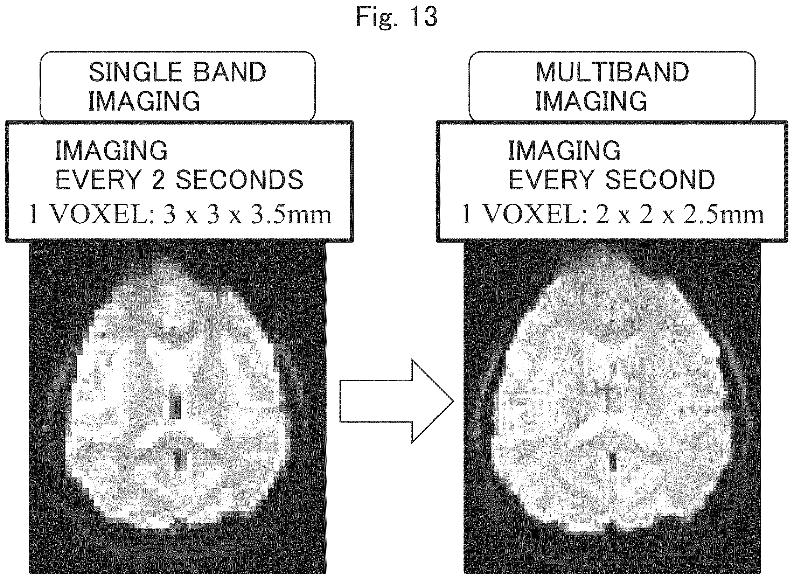

Fig. 13 shows results of imaging by single-band imaging as compared with multi-band imaging.

By single-band imaging, by way of example, volume of one voxel is $3 \times 3 \times 3.5$ mm and imaging is done every two seconds. In contrast, by multiband imaging (for example, simultaneous imaging of six slices), volume of one voxel is $2 \times 2 \times 2.5$ mm and imaging can be done every second.

Spatial resolution and temporal resolution are generally in the trade-off relation. The spatial resolution is adjusted to be equal to or higher than a prescribed value by adjusting the number of cross-sections re-configured by simultaneous excitation and simultaneous collection. Here, the spatial resolution being "equal to or higher than a prescribed value" means that the resolution may correspond to the voxel volume of $2 \times 2 \times 2.5$ mm or smaller.

(Functional Mapping)

As the "functional connectivity," in the biomarker as described above, the functional connectivity is extracted as time correlation of activities between anatomically determined brain areas.

In the neurofeedback training also, it is possible to realize the functional connectivity as the object of training based on the activities in such anatomically determined brain areas.

On the other hand, it is known that regions attaining specific functions exist locally. Anatomical brain areas, however, are specified based only on the shape/structure (structure of wrinkles and the like), and regions attaining specific functions differ person to person.

Therefore, as the "functional connectivity as the object of training," in the following, regions actually attaining a specific function are identified in an anatomical brain area and its vicinity of each individual, and the connectivity is extracted as time correlation of activities between the regions thus identified.

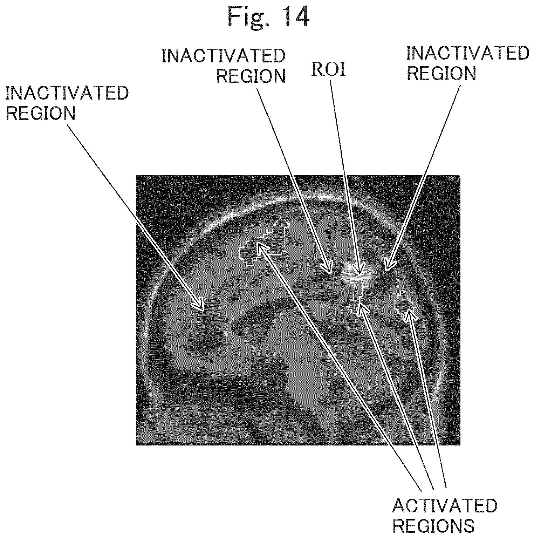

Fig. 14 shows relations between an anatomical region of interest (ROI) of brain areas and regions activated with respect to a specific function.

As shown in Fig. 14, there is a difference between an anatomical region of interest (ROI) and regions that are activated or inactivated while the subject is caused to do a specific task.

Assume that the above-described "functional connectivity with left Dorsolateral Prefrontal Cortex (L-DLPFC) with left Precuneus (L- Precuneus) and left posterior cingulated cortex (L-PCC)", which corresponds to ID = 1 above, is adopted as the "functional connectivity as the object of training." These regions are known as regions that are activated or inactivated by a specific working memory task or tasks.

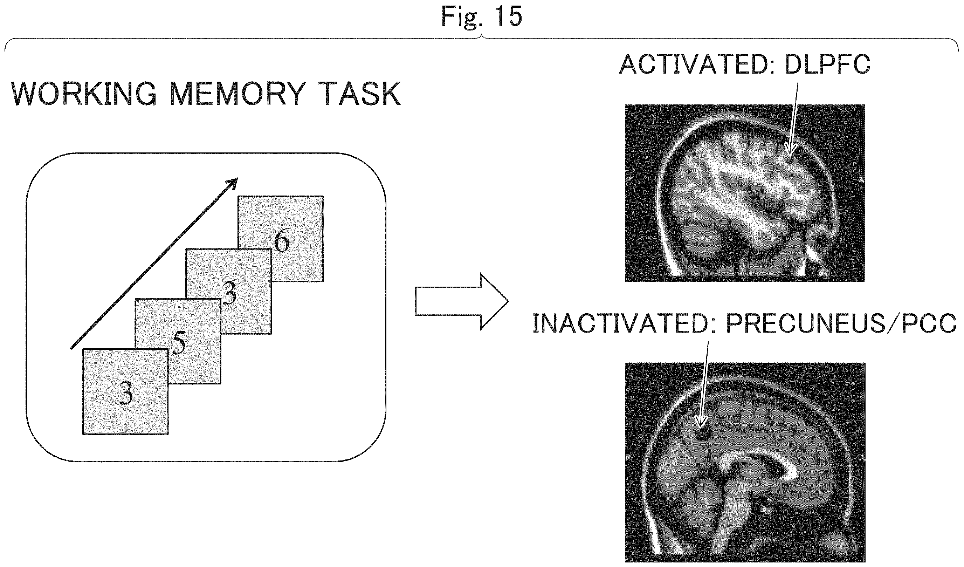

Fig. 15 shows a working memory task and relation between activation/inactivation of brain regions.

As a working memory task, it is possible to specify a functional region by asking the subject to take a so-called "n-back task."

The n-back task is a continuous performance task often used when brain activities of participants of an experiment are to be studied in the field of brain imaging or in psychological tests.

A series of stimuli is presented to a participant of an experiment, and the participant answers whether the currently presented stimulus is the same as the one presented N-times earlier. The difficulty level of the task is adjusted by the load factor N.

By way of example, in a visual 2-back task, a system presents the following numerals to a participant of an experiment.

353612 ...

The subject must respond by pressing, for example, a button when "3" in the sequence above is presented for the second time, since it is the same as the number presented two times earlier.

That the activity of "left Dorsolateral Prefrontal Cortex (DLPFC)" is activated while that of "left Precuneus and posterior cingulated cortex (PCC)" are inactivated by such a working memory task is disclosed, for example, in the following reference.

Reference: Genevieve Rayner, Graeme Jackson, Sarah Wilsona, "Cognition-related brain networks underpin the symptoms of unipolar depression: Evidence from a systematic review", Neuroscience and Biobehavioral Reviews 61 (2016) 53-65.

A subject having symptoms of depression tends to have weaker activation than healthy people in left Dorsolateral Prefrontal Cortex and weaker inactivation in left Precuneus. Even such a subject, however, has the same directions of activation and inactivation as healthy people.

Therefore, having a subject execute such a working memory task before executing the neurofeedback, it becomes possible to specify "functionally mapped brain regions" that correspond to anatomical brain areas.

Here, anatomical brain areas can be specified by picking up structural images before starting training of one day. By identifying an overlapping region of "functionally mapped brain regions" specified by the working memory task as described above and the anatomical brain areas on the structural images, it is possible to select a functionally more homogeneous region for each individual subject from the same anatomical brain areas as the "target brain region for training." Information for specifying such a "target brain region for training" is stored in a storage of a system and the same region is used as the object of training throughout the training. The "target region for training" is not limited to the "overlapping region" mentioned above and the "functionally mapped brain region" itself may be used.

In the neurofeedback based on the correlation of activities in the "target region for training" specified in this manner, the score to be fedback is calculated in a manner as described later.

(Details of Neurofeedback Training Sequence)

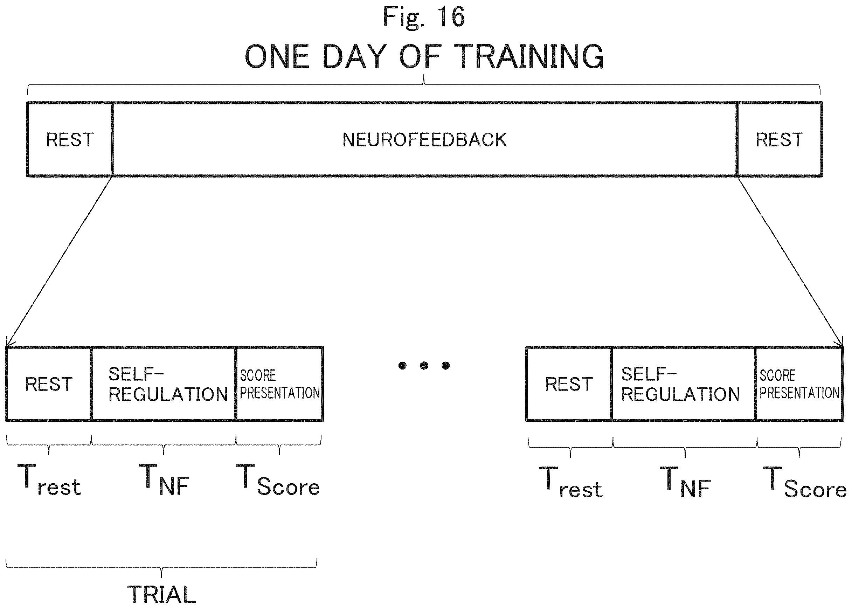

Fig. 16 is an illustration showing details of the neurofeedback training sequence.

Before and after the neurofeedback training, brain activities in the resting state are imaged (measured). As described above, this is to evaluate the effect of neurofeedback training. For instance, based on the result of measurements, it is possible to calculate biomarker values and to evaluate the effects of training.

As described with reference to Fig. 12, neurofeedback is done for a number of days.

The period of neurofeedback training of one day includes a plurality of neurofeedback training blocks referred to as "trials." Though not limited, a total of about 20 to 60 trials are performed per day.

In each trial, first, an instruction to start the resting period is given to the subject by displaying visual information on a display 6. In each trial, the subject is requested to be in a resting state without imagining anything for a time period $T_{rest}$ (hereinafter referred to as a "resting period"). Thereafter, an instruction to start a period of "self-regulation" (hereinafter referred to as "self-regulation period") is given, for example, by visual information.

It is assumed that an instruction to imagine something in the self-regulate period to make the score higher (larger) in accordance with feedback score information to be presented, was given in advance to the subject.

It is not particularly necessary to instruct beforehand what to imagine in the self-regulation period, and each subject repeats trial and error to find a state that increases the score. It may be possible, however, that the subject may imagine a task designated during the "self-regulation" of day 1. Alternatively, based on information obtained by hearing from other subjects, a plurality of possibilities that could have contributed to higher scores may be presented to the subject beforehand, and the subject may try to imagine such possible objects. In any event, there is no such condition that imagining something always increases the score for any subject. Therefore, the object of self-regulation has to be found by the subject through his/her trial and error.

The self-regulation period is time $T_{NF}$.

Further, in a score presenting period following the time $T_{NF}$, based on the correlation of activities between the "target brain regions for training" during the self-regulating period, information related to the score value calculated in a manner described below is presented as feedback score information to the subject. The time period in which the score is presented is time $T_{SCORE}$.

When presentation of the score ends, an instruction to start the resting period is given to the subject by a display on display 6.

Though not specifically limited, the time $T_{rest}$ is about 10 to about 20 seconds, the time $T_{NF}$ is about 30 to about 60 seconds, and the time $T_{SCORE}$ is about 10 to about 20 seconds.

(Trial Scheme and Score Calculation in Neurofeedback)

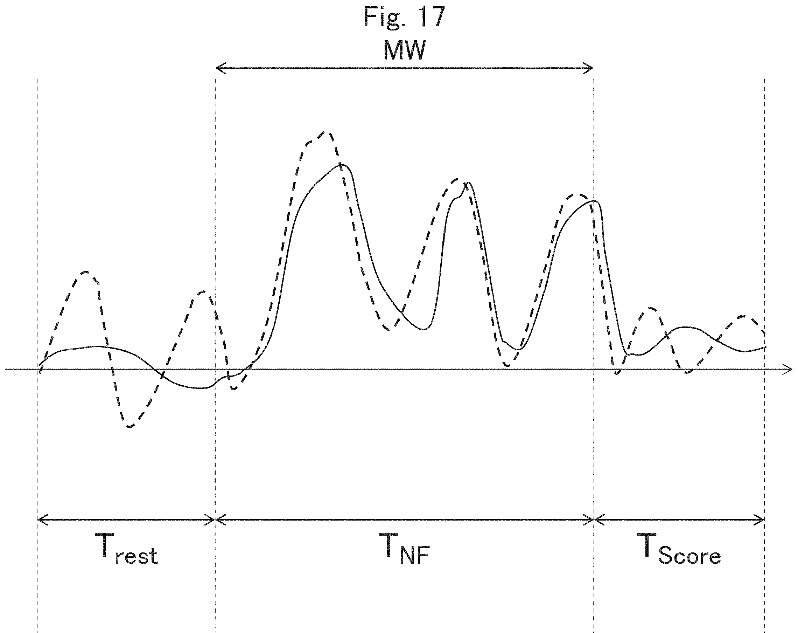

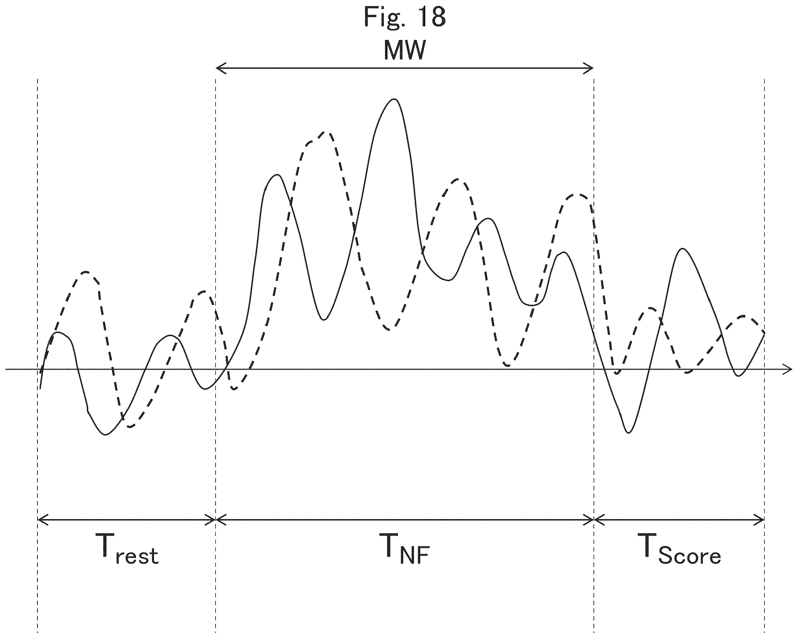

Figs. 17 and 18 show changes over time in brain activities in "target brain regions" (distinguished by reference characters FMR1 and FMR2 and simply referred to as "brain region FMR1" and "brain region FMR2." Both are generally referred to as "target brain region FMR for training.") in a trial period shown in Fig. 17. By way of example, "brain region FMR1" and "brain region FMR2" correspond to functional connectivity (ID = 1).

In Figs. 17 and 18, the self-regulation period is also referred to as a measurement window MW.

The brain activities of the two target brain regions FMRs for training are respectively plotted by a dotted line and a solid line.

Referring to Fig. 17, both the brain activity of brain region FMR1 plotted by the solid line and the brain activity of brain region FMR2 plotted by the dotted line, in average, have higher levels in the self-regulation period than in the resting period. Further, the brain activities of brain regions FMR1 and FMR2 change over time substantially in phase in the self-regulation period.

In Fig. 18 also, both the brain activity of brain region FMR1 plotted by the solid line and the brain activity of brain region FMR2 plotted by the dotted line, in average, have higher levels in the self-regulation period than in the resting period. The brain activities of brain regions FMR1 and FMR2, however, change with time substantially in opposite phases in the self-regulation period.

Here, data processing unit 102 calculates, from fMRI data (signals) of continuous n measurement time points (n: natural number, n ≥ 1) of fMRI in the resting state measured on real-time basis, the "degree of activity" of the target brain region FMR for training. For example, the degree of activities may be an average of measurements at continuous n measurement time points. Here, the period comprised of continuous n measurement time points is referred to as a "sample step."

(Method of Calculating Typical Correlation Coefficient)

First, as a premise of describing the method of calculating the correlation coefficient of the present embodiment, let us assume that the following "typical method of calculating time correlation" as below is used, for calculating the correlation on time-axis of the degree of activities in a measurement window of a prescribed time period shown in Figs. 17 and 18 in connection with the degree of activities at respective sample steps.

It is noted that the measurement window is such a time period that includes the degrees of activities of a plurality of sample steps continuous on the time axis (for example, m steps (m: natural number, m ≥ 2).

[Expression 10]

$$\frac{\sum (x - \bar{x})(y - \bar{y})}{\sqrt{\sum (x - \bar{x})^2 \sum (y - \bar{y})^2}}$$

Here, x represents the "degree of activity" of one brain region FMR1 of the "training target functional connectivities" and y represents the "degree of activity" of the other brain region FMR2 of the "training target functional connectivities." Further, x(bar) (hereinafter, the sign representing "mean value" by adding a bar (-) over a letter x will be referred to as "x(bar)") represents a mean value of the degree of activity x in the measurement window mentioned above. The same applies to "y(bar)". In the formula above, Σ represents the summation of the degrees of activities of m sample steps, for example, in the measurement window.

When the time correlation is calculated in accordance with the formula above, in both examples shown in Figs. 17 and 18, the level of brain activities in average increases in the self-regulation period than in the resting period, while the time correlation of the functional connectivities assumes, as a sign, a positive or negative value in accordance with whether the change in the brain activity level is in phase or in opposite phase.

(Method of Calculating Correlation Coefficient of the Present Embodiment)

In contrast, in the present embodiment, we define a correlation value $Cor_i$ (sample correlation) of the i-th trial i in a training of one day by the equation below.

[Expression 11]

$$Cor_i = \frac{\sum_{j=1}^{m} (x_j - \bar{x}_{res-1})(y_j - \bar{y}_{res-1})}{\sqrt{\sum_{j=1}^{m} (x_j - \bar{x}_{res-1})^2} \sqrt{\sum_{j=1}^{m} (y_j - \bar{y}_{res-1})^2}}$$

Here, $x_{res-i}$ (bar) is not a mean value of the measured values (BOLD signal level) of the brain activities in the measurement window but a mean value of the measurement values of brain activities in the resting period $T_{rest}$ in trial i. The same applies to $y_{res-i}$ (bar).

Specifically, data processing unit 102 first calculates the "degree of activities" of each of the brain regions FMR1 and FMR2 from the fMRI data (BOLD signal levels) of n continuous measurement time points (n: natural number, n ≥ 1) of fMRI in the resting state measured on the real-time basis, as described above. By way of example, the degree of activity may be a mean value of measurement values at n continuous measurement time points. Here again, the time period comprised of continuous n measurement time points will be referred to as a "sample step." The degrees of activities in the j-th sample step in the self-regulation period will be denoted by $x_j$ and $y_j$, respectively.

For the degrees of activities of the respective sample steps, data processing unit 102 calculates the time correlation of degrees of activities in accordance with the formula representing the correlation value Cor$_i$ described above for the measurement window of a prescribed time period. Here again, the measurement window includes the degrees of activities of a plurality of sample steps continuous on the time axis (for example, m steps (m: natural number, m $\geq$ 2)).

As described above, in calculating the time correlation, when a sample correlation coefficient is to be calculated from time data sequence of measured degrees of activities, sample covariance s$_{xy}$ as the numerator of the formula of correlation coefficient Cor$_i$, and sample standard deviations s$_x$, s$_y$, in respective root signs in the denominator of sample correlation coefficient, are calculated with the value (arithmetic average) of average degree of activity to be subtracted from the value of each data of degree of activity being not the arithmetic average of data in the measurement window period but the arithmetic average of data of the degree of activity in the resting period immediately preceding the measurement window.

With this approach, the correlation value becomes positive both in the example of Fig. 17 in which the change is in phase and in the example of Fig. 18 in which the change is in opposite phase. In other words, it becomes possible to calculate "time correlation" that reflects changes over time in the global brain activities in the measurement window.

Specifically, the approach above means that the level as a baseline for the degree of activity in each of the brain regions FMR1 and FMR2 is calculated from the degree of activity in the resting period, and the time correlation of brain activities of brain regions FMR1 and FMR2 is calculated in the self-regulation period following the resting period.

If there are a plurality of brain function connectivities as the target of training, the above-described time correlation is calculated for respective pairs of brain regions that correspond to the plurality of brain function connectivities.

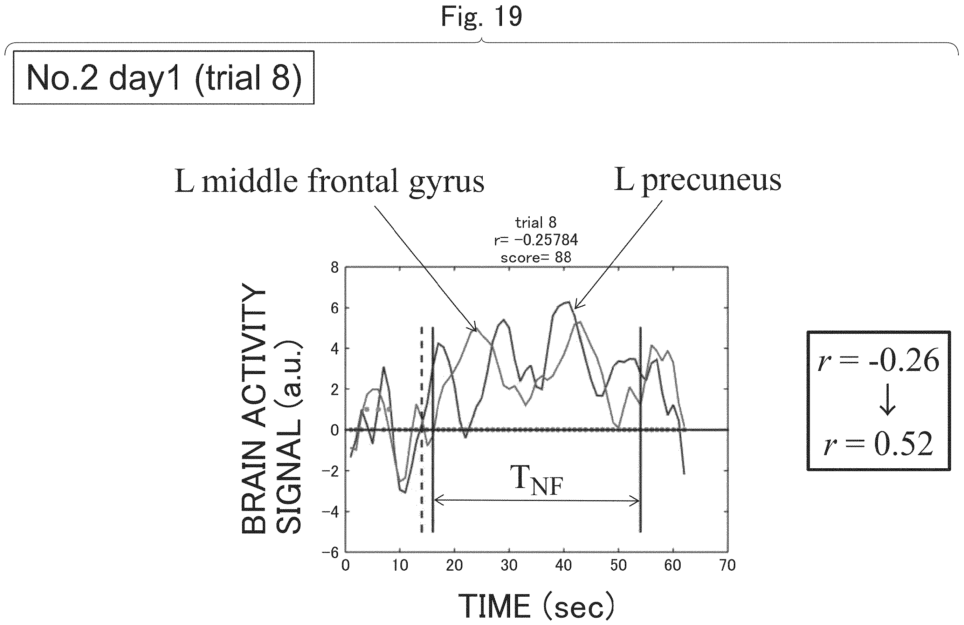

Figs. 19, 20, 21 and 22 show changes over time in actually measured brain activity signal indicating functional connectivity of a certain subject.

Figs. 19, 20, 21 and 22 show changes over time in brain activity signals corresponding to "functional connectivity between left Dorsolateral Prefrontal Cortex (DLPFC) and left Precuneus and left posterior cingulated cortex (PCC)".

In Fig. 19, the value of the "typical correlation coefficient" described above was r = - 0.26, while the value of the correlation coefficient in accordance with the present embodiment is r = 0.52.

It can be seen that the global correlation (positive correlation) of the changes over time in brain activities is reflected.

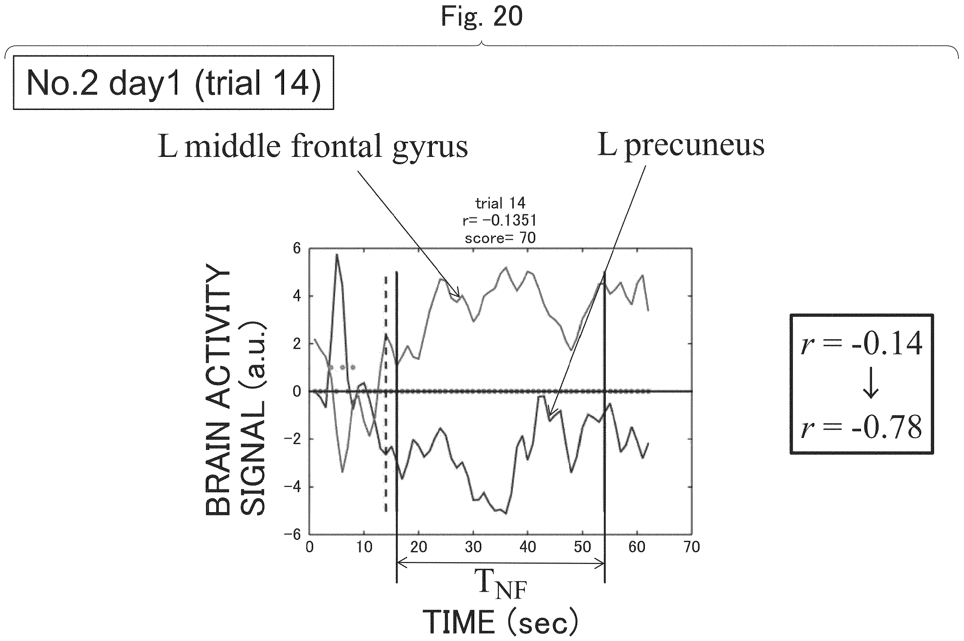

Similarly, in Fig. 20, the value of the "typical correlation coefficient" described above was r = - 0.14, while the value of the correlation coefficient in accordance with the present embodiment is r = - 0.78.

It can also be seen that the global correlation (negative correlation) of the changes over time in brain activities is reflected.

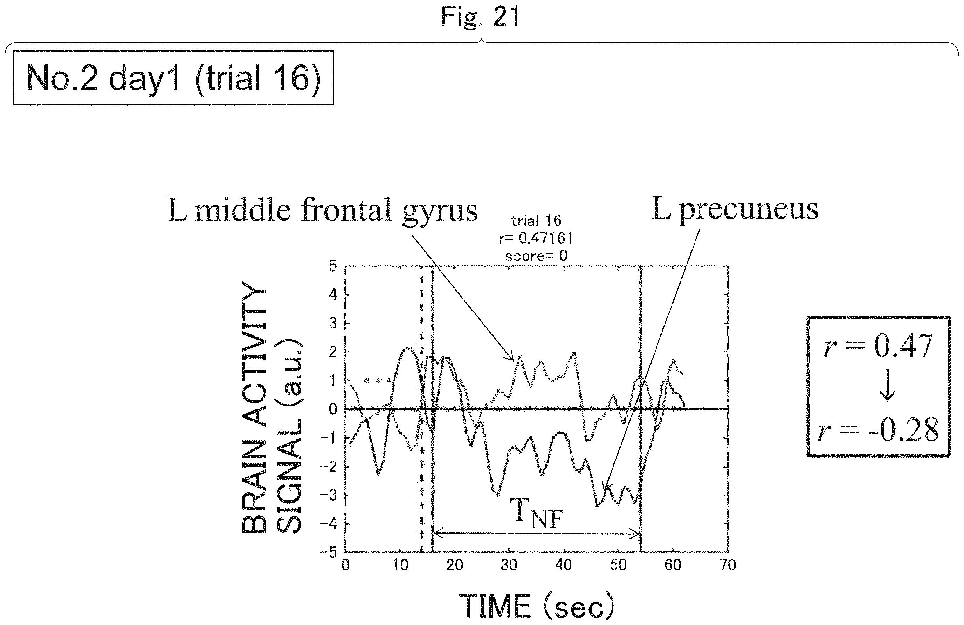

In Fig. 21, the value of the "typical correlation coefficient" described above was r = 0.47 while the value of the correlation coefficient in accordance with the present embodiment is r = - 0.28.

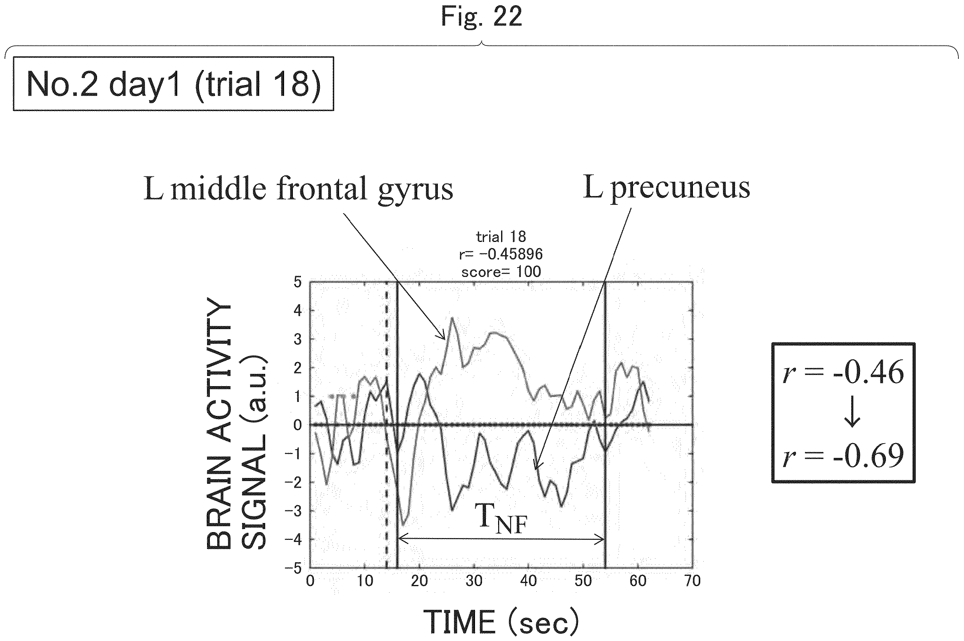

In Fig. 22, the value of the "typical correlation coefficient" described above was r = - 0.46, while the value of the correlation coefficient in accordance with the present embodiment is r = - 0.69.

(Feedback Score Information Calculating Process)

In the period of neurofeedback training, data processing unit 102 calculates the feedback score such that the feedback score is 50 when the functional connectivity as the object of training (time correlation value of the degree of activity of the target brain region) is the "baseline correlation value," and the score is 100 when the functional connectivity attains to the target value of the brain function connectivity ("target correlation value Cor$_T$").

Specifically, the baseline correlation value Cor$_B$ is the mean value of the time correlation values of the degrees of activities of the target brain region measured on day 1 shown in Fig. 12, and this value is fixed during training of days 2 to 5.

Further, the target correlation value Cor$_T$ is obtained by reverse z transformation of "mean value - 1SD" to be the time correlation value as described above. Therefore, it follows that the correlation value when it attains [(mean value) - 1 $\times$ (variance)] as the estimated period for the baseline correlation value is set as the target correlation value Cor$_T$.

Here, using the correlation value Cor$_i$ of trial i, the score value Score$_i$ to be fedback to the subject is calculated in accordance with the equation below.

In other words, the degree of approximation to the brain activity of healthy people is represented by the equation below.

[Expression 12]

$$Score_i = 50 \times \left(1 + \frac{Cor_i - Cor_B}{Cor_T - Cor_B}\right)$$

In the equation above, when the correlation value $Cor_i$ calculated in trial i is equal to the baseline correlation value $Cor_B$, the score becomes 50, and when the correlation value $Cor_i$ attains to "mean value -1SD", the score becomes 100.

The equation representing the degree of approximation of the subject's brain activity to that of healthy people is not limited to the equation above. For example, as the target correlation value $Cor_T$, generally, it may be set to a value corresponding to [(mean value) - a $\times$ (variance)] (a: constant not smaller than 1) after z transformation. Further, the score when the correlation value is equal to the baseline correlation value, or the score when it is equal to the target correlation value may be set to a different value.

If the change in z correlation value in the positive direction means approximation closer to the state of healthy people, in period estimation, the target correlation value $Cor_T$ may be "mean value + 1 SD." More generally, the sign of the value to be multiplied by the variance (sign preceding the constant a) is made positive and the target correlation value $Cor_T$ may be calculated as [(mean value) + a $\times$ (variance)].

Generally, the sign of time correlation of functional connectivity may possibly be negative for healthy people while it is positive for a subject determined to have "depression symptoms." Naturally, the opposite situation may be possible, and both may have the same sign. Here, the sign of the time correlation of functional connectivity and its absolute value magnitude are collectively referred to as "time correlation pattern."

The "degree of approximation" is not limited to the example above, and any other function may be used provided that as the time correlation pattern including the sign becomes closer to the time correlation pattern of healthy people, the "degree of approximation" becomes higher.

Though not limiting, if two or more "training target functional connectivities" are selected, an average of respective degrees of approximation may be used as the "degree of approximation," or a weighted average of the degrees of approximation with contribution of each connectivity may be used as the "degree of approximation."

Further, data processing unit 102 generates a diagram shape representing a reward value to be fedback in accordance with the score (degree of approximation) calculated in the above-described manner, and displays it on display unit 6. By way of example, here, the diagram representing the reward value is generated to have a larger two-dimensional size as the degree of approximation becomes higher, in accordance with the degree of approximation to the time correlation pattern of the target functional connectivity.

Another implementation of the present embodiment includes a computer program that executes the process above to drive a brain activity training apparatus. Such a program may be stored in a storage medium including hard disk, a semiconductor memory device such as a flash memory or an optical disk. The format of storing the program in the storage medium is not limited as long as the presenting device can read the program. Non-volatile storage in the storage medium is preferred. Alternatively, the program may be downloaded through a network.

As described above, by using the connectivity neurofeedback of the present embodiment, it is possible to use the correlation of connectivity between brain areas measured by real time fMRI for feedback information and thereby to change the correlation of connectivity between the brain areas through training.

Here, as disclosed in Non-Patent Literature 11 listed above, there is a report that by the connectivity neurofeedback, Hamilton Depression Rating Scale (HAM-D) of a "subject exhibiting depression symptoms" was improved after the neurofeedback than before.

In the foregoing description, it is assumed that real time fMRI is used as the brain activity detecting device for time-sequentially measuring brain activities by functional brain imaging. It is noted, however, that any of the fMRI described above, a magnetoencephalography, a near-infrared spectroscopy (NIRS), an electroencephalography or any combination of these may be used as the brain activity detecting device. Regarding such a combination, it is noted that fMRI and NIRS detect signals related to changes in blood stream in the brain, and have a high spatial resolution. On the other hand, magnetoencephalography and electroencephalography are characterized in that they have a high temporal resolution, for detecting changes in an electromagnetic field accompanying the brain activities. Therefore, if fMRI and the magnetoencephalography are combined, brain activities can be measured with both spatially and temporally high resolutions. Alternatively, by combining NIRS and the electroencephalography, a system for measuring the brain activities with both spatially and temporally high resolutions can also be implemented in a small, portable size.

Further, other methods may be utilized to execute the connectivity neurofeedback by using, for example, the method described in the reference below, in which components corresponding to connectivity between brain areas as the target of training are extracted based on measurement signals from a plurality of channels distributed spatially on the surface side of one's head, in measurement of NIRS or multi-channel electroencephalograph mounted on the subject's head.

Reference: Jun-ichiro Hirayama, Aapo Hyvarinen, Motoaki Kawanabe, "SPLICE: Fully Tractable Hierarchical Extension of ICA with Pooling", ICML 2017:1491-1500

By the configuration as described above, it becomes possible, by utilizing correlation of connectivity between brain areas measured by functional brain imaging as feedback information, to realize a brain activity training apparatus that changes the correlation of connectivity between brain areas.

Further, in the foregoing, an example has been described in which a "diagnosis label" is included as an attribute of a subject, and by generating a discriminator through machine learning, the discriminator is caused to function as a biomarker. The present invention, however, is not necessarily limited to such an example. Provided that subjects are classified into classes by an objective method before their measurements for applying machine learning are obtained, that the correlation of degrees of activities (connection) among brain regions (ROIs) of the subjects may be measured and that a discriminator can be generated for classification by machine learning using the measured results, the present invention may be used for other discrimination.

Therefore, it is possible to objectively pre-evaluate whether a specific "training pattern" by the brain activity training apparatus is effective to improve the health of a subject or not, and using this, to realize a brain activity training apparatus for improving health. Further, before the onset of a disease (in the state of "not yet diseased"), it is possible to objectively evaluate beforehand whether a specific "training pattern" by the brain activity training apparatus is helpful toward better health of a subject or not, and using this, to realize a brain activity training apparatus for promoting health.

The embodiments as have been described here are mere examples and should not be interpreted as restrictive. The scope of the present invention is determined by each of the claims with appropriate consideration of the written description of the embodiments and embraces modifications within the meaning of, and equivalent to, the languages in the claims.

INDUSTRIAL APPLICABILITY

The brain function improvement assisting apparatus in accordance with the present invention is applicable to autonomous training, cognitive learning, rehabilitation, sports relaxation and learning to adapt to an environment for attaining better brain state.

REFERENCE SIGNS LIST

2       subject
6       display
10      MRI apparatus
11      magnetic field applying mechanism
12      static magnetic field generating coil
14      magnetic field gradient generating coil
16      RF irradiating unit
18      bed
20      receiving coil
21      driving unit 21
22      static magnetic field power source
24      magnetic field gradient power source
26      signal transmitting unit
28      signal receiving unit
30      bed driving unit
32      data processing unit
36      storage unit
38      display unit
40      input unit
42      control unit
44      interface unit
46      data collecting unit
48      image processing unit
50      network interface

**Claims**

1.  A brain activity training apparatus for executing a neurofeedback training, comprising:

    a storage device (36) storing information for specifying, based on signals representing brain activities at a plurality of prescribed regions in a brain of each of a plurality of first and second subjects time-sequentially measured in advance, a functional connectivity as an object of training from at least one functional connectivity selected by a feature selection for determining a state of a prescribed functional connectivity through machine learning, from functional connectivities among said plurality of prescribed regions; wherein

said first subject belongs to a first group having a first pattern of time correlation of functional connectivity serving as a target pattern, and said second subject belongs to a group having a second pattern of time correlation of functional connectivity different from said target pattern;

said apparatus further comprising:

a brain activity detecting device for time-sequentially detecting signals representing brain activities at a plurality of brain regions in a brain of a trainee of said neurofeedback training, the plurality of brain regions in the brain of said trainee corresponding to respective ones of the plurality of prescribed regions in the brains of said first and second subjects;

a presenting device (38); and

a processing device (42); wherein

said neurofeedback training is done for a number of days and involves repetition of a plurality of trials, each trial including a resting period, a self-regulation period following the resting period and a presenting period in which feedback information is presented, following the self-regulation period;

said processing device (42) is configured such that

i) from signals detected from said trainee in said self-regulation period in a first day of said number of days by said brain activity detecting device, a baseline level of degree of activity of each of said prescribed regions corresponding to the functional connectivity as the object of training is calculated,

ii) for said trainee, from the signals detected in said self-regulation period in said number of days other than the first day by said brain activity detecting device and from the baseline level, time correlation of degrees of activity of said prescribed regions corresponding to the functional connectivity as the object of training is calculated,

iii) based on the calculated time correlation, a reward value is calculated in accordance with a degree of approximation to said target pattern, and

iv) feedback information indicating magnitude of the reward value is presented by said presenting device to the trainee.

2. The brain activity training apparatus according to claim 1, wherein said degree of approximation is defined by such a function that the degree of approximation becomes higher as said time correlation of said self-regulation period becomes closer to a target time correlation.

3. The brain activity training apparatus according to claim 1 or 2, wherein

the plurality of prescribed regions in the brains of said first and second subjects are anatomically defined brain areas; and

the plurality of brain regions in the brain of said trainee corresponding to the functional connectivity as the object of training are regions specified in advance for each trainee as regions of which brain activity is activated or inactivated when a known task is executed for specifying said brain areas.

4. The brain activity training apparatus according to any of claims 1 to 3, wherein the functional connectivity as the object of training include a functional connectivity between left Dorsolateral Prefrontal Cortex and left Precuneus and left posterior cingulated cortex.

5. The brain activity training apparatus according to any of claims 1 to 4, wherein the first subject is a healthy person; and the second subject is a subject exhibiting symptoms of depression, and the symptoms of depression are those of melancholic depression.

6. A computer program comprising instructions for controlling the brain activity training apparatus according to any of claims 1 to 5 by:

a) controlling the storage device (36) to store information for specifying, based on signals representing brain activities at a plurality of prescribed regions in a brain of each of a plurality of first and second subjects time-sequentially measured in advance, a functional connectivity as an object of training from at least one functional connectivity selected by a feature selection for determining a state of a prescribed functional connectivity through machine learning, from functional connectivities among said plurality of prescribed regions; wherein said first subject belongs to a first group having a first pattern of time correlation of functional connectivity serving as a target pattern, and said second subject belongs to a group having a second pattern of time correlation of functional connectivity different from said target pattern;

b) further controlling the brain activity detecting device to time-sequentially detect signals representing brain activities at a plurality of brain regions in a brain of a trainee of said neurofeedback training, the plurality of brain regions in the brain of said trainee corresponding to respective ones of the plurality of prescribed regions in the brains of said first and second subjects;

c) further controlling the presenting device (38); and

the processing device (42); wherein

said neurofeed back training is done for a number of days and involves repetition of a plurality of trials, each trial including a resting period, a self-regulation period following the resting period and a presenting period in which feedback information is presented, following the self-regulation period;

said processing device and presenting device being controlled by the computer program such that

i) from signals detected from said trainee in said self-regulation period in a first day of said number of days by said brain activity detecting device, a baseline level of degree of activity of each of said prescribed regions corresponding to the functional connectivity as the object of training is calculated,

ii) for said trainee, from the signals detected in said self-regulation period in said number of days other than the first day by said brain activity detecting device and from the baseline level, time correlation of degrees of activity of said prescribed regions corresponding to the functional connectivity as the object of training is calculated,

iii) based on the calculated time correlation, a reward value is calculated in accordance with a degree of approximation to said target pattern, and

iv) feedback information indicating magnitude of the reward value is presented by said presenting device to the trainee.

## Patentansprüche

1. Einrichtung zum Trainieren der Gehirnaktivität, um ein Neurofeedback-Training auszuführen, aufweisend:

eine Speichervorrichtung (36), die Informationen speichert, um basierend auf Gehirnaktivitäten an einer Vielzahl vorgeschriebener Bereiche in einem Gehirn von jedem einer Vielzahl erster und zweiter Testpersonen repräsentierenden Signalen, die vorher zeitsequentiell gemessen wurden, eine funktionale Konnektivität als Trainingsgegenstand von zumindest einer funktionalen Konnektivität, die mittels einer Merkmalsauswahl ausgewählt wird, um einen Zustand einer vorgeschriebenen funktionalen Konnektivität durch maschinelles Lernen zu bestimmen, aus funktionalen Konnektivitäten unter der Vielzahl vorgeschriebener Bereiche zu spezifizieren; wobei

die erste Testperson zu einer ersten Gruppe gehört, die ein erstes Muster einer Zeitkorrelation einer funktionalen Konnektivität aufweist, das als Zielmuster dient, und die zweite Testperson zu einer Gruppe gehört, die ein zweites Muster einer Zeitkorrelation einer funktionalen Konnektivität aufweist, das sich vom Zielmuster unterscheidet;

wobei die Einrichtung ferner aufweist:

eine Gehirnaktivitäten detektierende Vorrichtung, um Signale zeitsequentiell zu detektieren, die Gehirnaktivitäten an einer Vielzahl von Gehirnbereichen in einem Gehirn eines Trainees des Neurofeedback-Trainings repräsentieren, wobei die Vielzahl von Gehirnbereichen im Gehirn des Trainees jeweiligen der Vielzahl vorgeschriebener Bereiche in den Gehirnen der ersten und zweiten Testpersonen entspricht;

eine Präsentationsvorrichtung (38); und

eine Verarbeitungsvorrichtung (42); wobei

das Neurofeedback-Training über eine Anzahl von Tagen durchgeführt wird und mit einer Wiederholung einer Vielzahl von Versuchen verbunden ist, wobei jeder Versuch eine Ruheperiode, eine Selbstregulierungsperiode nach der Ruheperiode und eine Präsentationsperiode, in der die Feedback-Informationen präsentiert werden, nach der Selbstregulierungsperiode umfasst;

die Verarbeitungsvorrichtung (42) so konfiguriert ist, dass

i) aus Signalen, die vom Trainee in der Selbstregulierungsperiode an einem ersten Tag der Anzahl von Tagen mittels der Gehirnaktivitäten detektierenden Vorrichtung detektiert werden, ein Ausgangspegel eines Grads der Aktivität von jedem der vorgeschriebenen Bereiche entsprechend der funktionalen Konnektivität als der Trainingsgegenstand berechnet wird,

ii) für den Trainee aus den Signalen, die in der Selbstregulierungsperiode in der Anzahl von Tagen außer dem ersten Tag mittels der Gehirnaktivitäten detektierenden Vorrichtung detektiert werden, und aus dem Ausgangspegel eine Zeitkorrelation von Graden der Aktivität der vorgeschriebenen Bereiche entsprechend der funktionalen Konnektivität als der Trainingsgegenstand berechnet wird,

iii) basierend auf der berechneten Zeitkorrelation ein Belohnungswert gemäß einem Grad einer Approximation an das Zielmuster berechnet wird und

iv) eine Feedback-Information, die die Größe des Belohnungswerts angibt, dem Trainee mittels der Präsentationsvorrichtung präsentiert wird.

2. Einrichtung zum Trainieren der Gehirnaktivität nach Anspruch 1, wobei der Grad einer Approximation durch solch eine Funktion definiert wird, dass der Grad einer Approximation umso höher wird, je näher die Zeitkorrelation der Selbstregulierungsperiode an eine Ziel-Zeitkorrelation kommt.

3. Einrichtung zum Trainieren der Gehirnaktivität nach Anspruch 1 oder 2, wobei

die Vielzahl vorgeschriebener Bereiche in den Gehirnen der ersten und zweiten Testpersonen anatomisch definierte Hirnareale sind; und

die Vielzahl von Gehirnbereichen in dem Gehirn des Trainees entsprechend der funktionalen Konnektivität als der Trainingsgegenstand Bereiche sind, die vorher für jeden Trainee als Bereiche spezifiziert wurden, deren Gehirnaktivität aktiviert oder deaktiviert wird, wenn eine bekannte Aufgabe ausgeführt wird, um die Hirnareale zu spezifizieren.

4. Einrichtung zum Trainieren der Gehirnaktivität nach einem der Ansprüche 1 bis 3, wobei die funktionale Konnektivität als der Trainingsgegenstand eine funktionale Konnektivität zwischen dem linken dorsolateralen präfrontalen Kortex und dem linken Precuneus und und dem linken posterioren cingulären Kortex umfasst.

5. Einrichtung zum Trainieren der Gehirnaktivität nach einem der Ansprüche 1 bis 4, wobei

die erste Testperson eine gesunde Person ist und

die zweite Testperson eine Person ist, Symptome einer Depression zeigt, und die Symptome einer Depression jene einer melancholischen Depression sind.

6. Computerprogramm, aufweisend Anweisungen, um die Einrichtung zum Trainieren der Gehirnaktivität nach einem der Ansprüche 1 bis 5 zu steuern, indem:

a) die Speichervorrichtung (36) gesteuert wird, um Informationen zu speichern, um basierend auf Gehirnaktivitäten an einer Vielzahl vorgeschriebener Bereiche in einem Gehirn von jedem einer Vielzahl erster und zweiter Testpersonen repräsentierenden Signalen, die vorher zeitsequentiell gemessen wurden, eine funktionale Konnektivität als Trainingsgegenstand von zumindest einer funktionalen Konnektivität, die mittels einer Merkmalsauswahl ausgewählt wird, um einen Zustand einer vorgeschriebenen funktionalen Konnektivität durch maschinelles Lernen zu bestimmen, aus funktionalen Konnektivitäten unter der Vielzahl vorgeschriebener Bereiche zu spezifizieren; wobei

die erste Testperson zu einer ersten Gruppe gehört, die ein erstes Muster einer Zeitkorrelation einer funktionalen Konnektivität aufweist, das als Zielmuster dient, und die zweite Testperson zu einer Gruppe gehört, die ein zweites Muster einer Zeitkorrelation einer funktionalen Konnektivität aufweist, das sich vom Zielmuster unterscheidet;

b) ferner die Gehirnaktivitäten detektierende Vorrichtung gesteuert wird, um Signale zeitsequentiell zu detektieren, die Gehirnaktivitäten an einer Vielzahl von Gehirnbereichen in einem Gehirn eines Trainees des Neurofeedback-Trainings repräsentieren, wobei die Vielzahl von Gehirnbereichen im Gehirn des Trainees jeweiligen der Vielzahl vorgeschriebener Bereiche in den Gehirnen der ersten und zweiten Testpersonen entspricht;

c) ferner die Präsentationsvorrichtung (38) und die Verarbeitungsvorrichtung (42) gesteuert werden; wobei das Neurofeedback-Training über eine Anzahl von Tagen durchgeführt wird und mit einer Wiederholung einer Vielzahl von Versuchen verbunden ist, wobei jeder Versuch eine Ruheperiode, eine Selbstregulierungsperiode nach der Ruheperiode und eine Präsentationsperiode, in der die Feedback-Informationen präsentiert werden, nach der Selbstregulierungsperiode umfasst;

wobei die Verarbeitungsvorrichtung und die Präsentationsvorrichtung durch das Computerprogramm so gesteuert werden, dass

i) aus Signalen, die vom Trainee in der Selbstregulierungsperiode an einem ersten Tag der Anzahl von Tagen mittels der Gehirnaktivitäten detektierenden Vorrichtung detektiert werden, ein Ausgangspegel eines Grads der Aktivität von jedem der vorgeschriebenen Bereiche entsprechend der funktionalen Konnektivität als der Trainingsgegenstand berechnet wird,

ii) für den Trainee aus den Signalen, die in der Selbstregulierungsperiode in der Anzahl von Tagen außer dem ersten Tag mittels der Gehirnaktivitäten detektierenden Vorrichtung detektiert werden, und aus dem Ausgangspegel eine Zeitkorrelation von Graden der Aktivität der vorgeschriebenen Bereiche entsprechend der funktionalen Konnektivität als der Trainingsgegenstand berechnet wird,

iii) basierend auf der berechneten Zeitkorrelation ein Belohnungswert gemäß einem Grad einer Approximation an das Zielmuster berechnet wird und

iv) eine Feedback-Information, die die Größe des Belohnungswerts angibt, dem Trainee mittels der Präsentationsvorrichtung präsentiert wird.

## Revendications

1. Appareil d'entraînement d'activité cérébrale pour exécuter un entraînement par rétroaction neurologique, comprenant :

un dispositif de stockage (36) stockant des informations pour spécifier, sur la base de signaux représentant des activités cérébrales au niveau d'une pluralité de régions prescrites dans le cerveau de chacun d'une pluralité de premier et deuxième sujets mesurées séquentiellement dans le temps à l'avance, une connectivité fonctionnelle en tant qu'objet d'entraînement à partir d'au moins une connectivité fonctionnelle sélectionnée au moyen d'une sélection de caractéristiques pour déterminer un état d'une connectivité fonctionnelle prescrite par apprentissage automatique, à partir de connectivités fonctionnelles parmi ladite pluralité de régions prescrites ; dans lequel
ledit premier sujet appartient à un premier groupe ayant un premier schéma de corrélation temporelle de connectivité temporelle servant de schéma cible, et ledit deuxième sujet appartient à un deuxième groupe ayant un deuxième schéma de corrélation temporelle de connectivité temporelle différent dudit schéma cible ;
ledit appareil comprenant en outre :

un dispositif de détection d'activité cérébrale pour détecter séquentiellement dans le temps des signaux représentant des activités cérébrales au niveau d'une pluralité de régions cérébrales dans le cerveau d'un apprenant dudit entraînement par rétroaction neurologique, la pluralité de régions cérébrales dans le cerveau dudit apprenant correspondant à des régions prescrites respectives parmi la pluralité de régions prescrites dans le cerveau desdits premier et deuxième sujets ;
un dispositif de présentation (38) ; et
un dispositif de traitement (42) ; dans lequel
ledit entraînement par rétroaction neurologique est effectué pendant un certain nombre de jours et implique la répétition d'une pluralité d'essais, chaque essai comportant une période de repos, une période d'autorégulation suivant la période de repos et une période de présentation pendant laquelle des informations de retour sont présentées, après la période d'autorégulation ;
ledit dispositif de traitement (42) est configuré de sorte que

i) à partir de signaux détectés en provenance dudit apprenant pendant ladite période d'autorégulation au cours d'un premier jour dudit nombre de jours par ledit dispositif de détection d'activité cérébrale, un niveau de base de degré d'activité de chacune desdites régions prescrites correspondant à la connectivité fonctionnelle en tant qu'objet d'entraînement soit calculé,
ii) pour ledit apprenant, à partir des signaux détectés pendant ladite période d'autorégulation au cours dudit nombre de jours autres que le premier jour par ledit dispositif de détection d'activité cérébrale et à partir du niveau de base, une corrélation temporelle de degrés d'activité desdites régions prescrites correspondant à la connectivité fonctionnelle en tant qu'objet d'entraînement soit calculée,

iii) sur la base de la corrélation temporelle calculée, une valeur de récompense soit calculée conformément à un degré de rapprochement avec ledit schéma cible, et

iv) des informations de retour indiquant une grandeur de la valeur de récompense sont présentées par ledit dispositif de présentation à l'apprenant.

2. Appareil d'entraînement d'activité cérébrale selon la revendication 1, dans lequel ledit degré de rapprochement est défini par une fonction telle que le degré de rapprochement augmente à mesure que ladite corrélation temporelle de ladite période d'autorégulation se rapproche d'une corrélation temporelle cible.

3. Appareil d'entraînement d'activité cérébrale selon la revendication 1 ou 2, dans lequel la pluralité de régions prescrites dans le cerveau desdits premier et deuxième sujets sont des zones cérébrales définies anatomiquement, et la pluralité de régions cérébrales dans le cerveau dudit apprenant correspondant à la connectivité fonctionnelle en tant qu'objet d'entraînement sont des régions spécifiées à l'avance pour chaque apprenant en tant que régions dont l'activité cérébrale est activée ou inactivée lorsqu'une tâche connue est exécutée pour spécifier lesdites zones cérébrales.

4. Appareil d'entraînement d'activité cérébrale selon l'une des revendications 1 à 3, dans lequel la connectivité fonctionnelle en tant qu'objet d'entraînement comporte une connectivité fonctionnelle entre le cortex préfrontal dorsolatéral gauche et le précunéus gauche et le cortex cingulaire postérieur gauche.

5. Appareil d'entraînement d'activité cérébrale selon l'une des revendications 1 à 4, dans lequel

le premier sujet est une personne en bonne santé ; et
le deuxième sujet est un sujet présentant des symptômes de dépression, et les symptômes de dépression sont ceux d'une dépression mélancolique.

6. Programme informatique comprenant des instructions pour commander l'appareil d'entraînement d'activité cérébrale selon
l'une des revendications 1 à 5 en :

a) commandant le dispositif de stockage (36) afin qu'il stocke des informations pour spécifier, sur la base de signaux représentant des activités cérébrales au niveau d'une pluralité de régions prescrites dans le cerveau de chacun d'une pluralité de premier et deuxième sujets mesurées séquentiellement dans le temps à l'avance, une connectivité fonctionnelle en tant qu'objet d'entraînement à partir d'au moins une connectivité fonctionnelle sélectionnée au moyen d'une sélection de caractéristiques pour déterminer un état d'une connectivité fonctionnelle par apprentissage automatique, à partir de connectivités fonctionnelles parmi ladite pluralité de régions prescrites ; dans lequel
ledit premier sujet appartient à un premier groupe ayant un premier schéma de corrélation temporelle de connectivité temporelle servant de schéma cible, et ledit deuxième sujet appartient à un deuxième groupe ayant un deuxième schéma de corrélation temporelle de connectivité temporelle différent dudit schéma cible ;
b) commandant en outre le dispositif de détection d'activité cérébrale pour détecter séquentiellement dans le temps des signaux représentant des activités cérébrales au niveau d'une pluralité de régions cérébrales dans le cerveau d'un apprenant dudit entraînement par rétroaction neurologique, la pluralité de régions cérébrales dans le cerveau dudit apprenant correspondant à des régions prescrites respectives parmi la pluralité de régions prescrites dans le cerveau desdits premier et deuxième sujets ;
c) commandant en outre le dispositif de présentation (38) ; et
le dispositif de traitement (42) ; dans lequel
ledit entraînement par rétroaction neurologique est effectué pendant un certain nombre de jours et implique la répétition d'une pluralité d'essais, chaque essai comportant une période de repos, une période d'autorégulation suivant la période de repos et une période de présentation dans laquelle des informations de retour sont présentées, après la période d'autorégulation ;
lesdits dispositifs de traitement et de présentation étant commandés par le programme informatique de sorte que

i) à partir de signaux détectés en provenance dudit apprenant pendant ladite période d'autorégulation au cours d'un premier jour dudit nombre de jours par ledit dispositif de détection d'activité cérébrale, un niveau de base de degré d'activité de chacune desdites régions prescrites correspondant à la connectivité fonctionnelle en tant qu'objet d'entraînement soit calculé,
ii) pour ledit apprenant, à partir des signaux détectés pendant ladite période d'autorégulation au cours dudit nombre de jours autres que le premier jour par ledit dispositif de détection d'activité cérébrale et à partir du niveau de base, une corrélation temporelle de degrés d'activité desdites régions prescrites correspondant à la connectivité fonctionnelle en tant qu'objet d'entraînement soit calculée,

iii) sur la base de la corrélation temporelle calculée, une valeur de récompense soit calculée conformément à un degré de rapprochement avec ledit schéma cible, et

iv) des informations de retour indiquant une grandeur de la valeur de récompense sont présentées par ledit dispositif de présentation à l'apprenant.

# Fig. 1

Fig. 2

32

2010

2030 DISK DRIVE

2080 HDD

2050

2070 RAM

2060 ROM

2040 CPU

2090 COMMUNICATION INTERFACE

2020 MEMORY DRIVE

2210

2120

2100

2110

2010

Fig. 3

Fig. 4

(CONVENTIONAL EXAMPLE)

(a)

(b)

## Fig. 5

TIME SEQUENTIAL
DATA OF rsfMRI

EXTRACT AVERAGE WAVEFORM
IN EACH REGION OF INTERST

CALCULATE CORRELATION MATRIX
AMONG 140 REGIONS

EP 3 763 288 B1

Fig. 6

CORRELATION MATRIX

(HEALTHY CONTOL/PATIENTS)

FEATURE EXTRACTION BY SCCA

FEATURE EXTRACTION BY SLR

DISCRIMINATOR BY SLR

Fig. 7

EP 3 763 288 B1

# Fig. 8

```
┌─────────────────────────────────────┐
│              START                   │
│   (GENERATION OF DISCRIMINATOR)      │
└─────────────────────────────────────┘
                  │  ⌐S100
                  ▼
┌─────────────────────────────────────┐
│         READ rs-fcMRI DATA           │
└─────────────────────────────────────┘
                  │  ⌐S102
                  ▼
┌─────────────────────────────────────┐
│ FEATURE EXTRACTION BY SPARSE         │
│ CANONICAL CORRELATION ANALYSIS       │
│ (INNER LOOP)                         │
└─────────────────────────────────────┘
                  │  ⌐S104
                  ▼
┌─────────────────────────────────────┐
│ FEATURE EXTRACTION BY SPARSE         │
│ LOGISTIC REGRESSION (OUTER LOOP)     │
└─────────────────────────────────────┘
                  │  ⌐S106
                  ▼
┌─────────────────────────────────────┐
│    GENERATION OF DISCRIMINATOR       │
└─────────────────────────────────────┘
                  │  ⌐S108
                  ▼
┌─────────────────────────────────────┐
│               END                    │
└─────────────────────────────────────┘
```

## Fig. 9

SELECT {

| ID | NAME | LAT. | BSA ATLAS (SULCUS) | BA | r CONTROL | r MDD | WEIGHT |
|---|---|---|---|---|---|---|---|
| 1 | PRECUNEUS/POSTERIOR CINGULATE CORTEX | L | INTERNAL PARIETAL SULCUS | 7, 23, 31 | −0.063 | 0.121 | 3.88 |
| | MIDDLE FRONTAL GYRUS, DORSOLATERAL PREFRONTAL CORTEX: DLPFC | L | INTERMEDIATE FRONTAL SULCUS | 46 | | | |
| 2 | SUPPLEMENTARY MOTOR AREA: SMA, PRE-SMA, FRONTAL EYE FIELDS, DORSOMEDIAL PREFRONTAL CORTEX | R | MEDIAN FRONTAL SULCUS | 6, 8, 9 | 0.175 | −0.017 | −3.34 |
| | INFERIOR FRONTAL GYRUS OPERCULAR PART | L | DIAGONAL RAMUS OF THE LATERAL FISSURE | 44 | | | |
| 3 | THALAMUS | L | THALAMUS | – | 0.210 | 0.051 | −2.61 |
| | ANTERIOR CINGULATE CORTEX, POSTERIOR CINGULATE CORTEX | R | SUBCALLOSAL SULCUS | 23, 24, 33 | | | |
| 4 | PRECUNEUS | L | SUPERIOR PARIETAL SULCUS | 7 | −0.155 | 0.014 | 1.99 |
| | INFERIOR FRONTAL GYRUS OPERCULAR PART | L | DIAGONAL RAMUS OF THE LATERAL FISSURE | 44 | | | |
| 5 | INFERIOR FRONTAL GYRUS OPERCULAR PART | R | INFERIOR PRECENTRAL SULCUS | 44 | 0.408 | 0.288 | −2.38 |
| | INFERIOR FRONTAL GYRUS TRIANGULAR PART | L | INFERIOR FRONTAL SULCUS | 45 | | | |
| 6 | NUCLEUS ACCUMBENS | R | ACCUMBENS | – | 0.010 | 0.134 | 2.22 |
| | ANTERIOR CINGULATE CORTEX, POSTERIOR CINGULATE CORTEX | R | SUBCALLOSAL SULCUS | 23, 24, 33 | | | |
| 7 | LINGUAL GYRUS | L | ANTERIOR INTRALINGUAL SULCUS | 18 | 0.074 | 0.163 | 2.75 |
| | MIDDLE OCCIPITAL GYRUS | R | LOBE OCCIPITAL | 19 | | | |
| 8 | POSTCENTRAL GYRUS (GUSTATORY AREA) | R | CENTRAL SYLVIAN SULCUS | 43 | −0.137 | −0.004 | 1.73 |
| | OCCIPITAL LOBE (VISUAL AREA) | L | LOBE OCCIPITAL | 17, 18, 19 | | | |
| 9 | SUPERIOR PARIETAL GYRUS (SOMATOSENSORY AREA) | L | SUPERIOR POSTCENTRAL SULCUS | 5 | 0.076 | −0.022 | −1.93 |
| | INFERIOR TEMPORAL GYRUS, FUSIFORM GYRUS | L | MEDIAN OCCIPITO-TEMPORAL LATERAL SULCUS | 20, 37 | | | |
| 10 | ROLANDIC OPERCULUM, SUPRAMARGINAL GYRUS (AUDITORY AREA) | R | POSTERIOR LATERAL FISSURE | 40, 41, 48 | 0.066 | 0.168 | 1.75 |
| | ORBITOFRONTAL CORTEX, INSULAR CORTEX, INFERIOR FRONTAL GYRUS ORBITAL PART | R | ANTERIOR LATERAL FISSURE | 12, 13, 47 | | | |
| 11 | OCCIPITAL LOBE (VISUAL ASSOCIATION AREA) | L | POSTERIOR INTRA-LINGUAL SULCUS | 18 | −0.144 | −0.052 | 1.59 |
| | ANTERIOR CINGULATE CORTEX, POSTERIOR CINGULATE CORTEX, PRECUNEUS (SOMATOSENSORY ASSOCIATION AREA) | L | CALLOSO-MARGINAL POSTERIOR FISSURE | 5, 7, 23, 24, 31, 33 | | | |
| 12 | SMA, PRE-SMA, FRONTAL EYE FIELDS, DLPFC | R | MEDIAN FRONTAL SULCUS | 6, 8, 9 | 0.207 | 0.115 | −1.37 |
| | ACC | L | CALLOSO-MARGINAL ANTERIOR FISSURE | 32 | | | |

EP 3 763 288 B1

# Fig. 10

1. EPI IMAGING   6. FEEDBACK

2. IMAGE RECONFIGURATION
(OUTPUT ON REAL-TIME BASIS)

TIME-SEQUENTIAL DATA

3. EXTRACT WAVEFORM

4. CALCULATE CORRELATION

$$Score_i = 50 \times \left(1 + \frac{Cor_i - Cor_B}{Cor_T - Cor_B}\right)$$

5. SCORING
(HIGHER SCORE FOR ONE CLOSER TO HEALTHY PEOPLE)

## Fig. 11

START OF TRAINING

TARGET VALUE

INDEX VALUE

TRAINEE SELF-REGULATE TO GET THE INDEX VALUE COME CLOSER TO TARGET VALUE

DURING TASK

TARGET VALUE

INDEX VALUE

EP 3 763 288 B1

Fig. 12

EP 3 763 288 B1

MEASUREMENT
OF ACTIVITIES
IN RESTING STATE

NEUROFEEDBACK
TRAINING

| PRELIMINARY RESTING STATE | → | TRAINING | → | TRAINING | → | TRAINING | → | TRAINING |

DAY 1　　　　DAY 2　　　　DAY 3　　　　DAY 4　　　　DAY 5

Fig. 13

SINGLE BAND IMAGING

IMAGING EVERY 2 SECONDS
1 VOXEL: 3 x 3 x 3.5mm

MULTIBAND IMAGING

IMAGING EVERY SECOND
1 VOXEL: 2 x 2 x 2.5mm

## Fig. 14

Fig. 15

WORKING MEMORY TASK

ACTIVATED: DLPFC

INACTIVATED: PRECUNEUS/PCC

Fig. 16

ONE DAY OF TRAINING

Fig. 17

MW

$T_{rest}$      $T_{NF}$      $T_{Score}$

Fig. 18

MW

$T_{Score}$

$T_{NF}$

$T_{rest}$

Fig. 19

No.2 day1 (trial 8)

L middle frontal gyrus          L precuneus

trial 8
r= −0.25784
score= 88

$r = -0.26$
↓
$r = 0.52$

BRAIN ACTIVITY SIGNAL (a.u.)

$T_{NF}$

TIME (sec)

EP 3 763 288 B1

Fig. 20

No.2 day1 (trial 14)

L middle frontal gyrus          L precuneus

trial 14
r= −0.1351
score= 70

$T_{NF}$

TIME (sec)

$r = -0.14$
↓
$r = -0.78$

Fig. 21

No.2 day1 (trial 16)

Fig. 22

No.2 day1 (trial 18)

L middle frontal gyrus          L precuneus

trial 18
r= −0.45896
score= 100

$r = -0.46$
↓
$r = -0.69$

TIME (sec)

BRAIN ACTIVITY SIGNAL (a.u.)

$T_{NF}$

EP 3 763 288 B1

## REFERENCES CITED IN THE DESCRIPTION

### Patent documents cited in the description

- WO 2006132313 A1
- WO 2014178323 A1
- WO 2017090590 A1
- WO 2014178322 A1
- US 2015294074 A1

### Non-patent literature cited in the description

- **ICHIKAWA N ; LISI G ; YAHATA N ; OKADA G ; TAKAMURA M ; YAMADA M ; SUHARA T ; HASHIMOTO R ; YAMADA T ; YOSHIHARA Y.** Identifying melancholic depression biomarker using whole-brain functional connectivity. *arXiv:1704.01039,* 2017
- **NIKOLAUS WEISKOPF.** Real-time fMRI and its application to neurofeedback. *NeuroImage,* 2012, vol. 62, 682-692
- **KATSUTOSHI MURATA.** Siemens sha-sei MR souchi no genjo to doukou. *MEDICAL IMAGING TECHNOLOGY,* January 2014, vol. 32 (1), 3-7
- **FEINBERG DA ; MOELLER S ; SMITH SM et al.** Multiplexed echo planar imaging for sub-second whole brain FMRI and fast diffusion imaging. *PLoS ONE,* 2010, vol. 5 (12), e15710
- **DECHARMS RC ; MAEDA F ; GLOVER GH et al.** Control over brain activation and pain learned by using real-time functional MRI. *Proc Natl Acad Sci USA,* 2005, vol. 102 (51), 18626-18631
- **RAICHLE ME ; MACLEOD AM ; SNYDER AZ.** A default mode of brain function. *Proc Natl Acad Sci USA,* 2001, vol. 98 (2), 676-682
- **TAL HARMELECH ; SON PREMINGER ; ELIAHU WERTMAN ; RAFAEL MALACH.** The Day-After Effect: Long Term, Hebbian-Like Restructuring of Resting-State fMRI Patterns Induced by a Single Epoch of Cortical Activation. *The Journal of Neuroscience,* 29 May 2013, vol. 33 (22), 9488-9497
- **KAZUHISA SHIBATA ; TAKEO WATANABE ; YUKA SASAKI ; MITSUO KAWATO.** Perceptual Learning Incepted by Decoded fMRI Neurofeedback Without Stimulus Presentation. *SCIENCE,* 09 December 2011, vol. 334
- **T. WATANABE ; J. E. NANEZ SR ; S. KOYAMA ; I.MUKAI ; J. LIEDERMAN ; Y. SASAKI.** Greater plasticity in lower-level than higher-level visual motion processing in a passive perceptual learning task. *Nature Neuroscience,* 2002, vol. 5, 1003-1009
- **NORIAKI YAHATA ; JUN MORIMOTO ; RYUICHIRO HASHIMOTO ; GIUSEPPE LISI ; KAZUHISA SHIBATA ; YUKI KAWAKUBO ; HITOSHI KUWABARA ; MIHO KURODA ; TAKASHI YAMADA ; FUKUDA MEGUMI.** A small number of abnormal brain connections predicts adult autism spectrum disorder. *NATURE COMMUNICATIONS*
- **TAKASHI YAMADA ; RYU-ICHIRO HASHIMOTO ; NORIAKI YAHATA ; NAHO ICHIKAWA ; YUJIRO YOSHIHARA ; YASUMASA OKAMOTO ; NOBUMASA KATO ; HIDEHIKO TAKAHASHI ; MITSUO KAWATO.** Resting-State Functional Connectivity-Based Biomarkers and Functional MRI-Based Neurofeedback for Psychiatric Disorders: A Challenge for Developing Theranostic Biomarkers. *International Journal of Neuropsychopharmacology,* 2017, vol. 20 (10), 769-781
- **SETSOMPOP K ; GAGOSKI BA ; POLOMENI JR.** Blipped-controlled aliasing in parallel imaging for simultaneous multislice echo planar imaging with reduced g-factor penalty. *Magn Reson Med,* 2012, vol. 67, 1210-1224
- **PERROT et al.** *Med Image Anal,* 2011, vol. 15 (4
- **TZOURIO-MAZOYER et al.** *Neuroimage,* 2002, vol. 15 (1
- **WITTEN DM ; TIBSHIRANI R ; T HASTIE.** A penalized matrix decomposition, with applications to sparse principal components and canonical correlation analysis. *Biostatistics,* 2009, vol. 10 (3), 515-534
- **OKITO YAMASHITA ; MASA AKI SATO ; TAKU YOSHIOKA ; FRANK TONG ; YUKIYASU KAMITANI.** Sparse Estimation automatically selects voxels relevant for the decoding of fMRI activity patterns. *NeuroImage,* 2008, vol. 42 (4), 1414-1429
- **GENEVIEVE RAYNER ; GRAEME JACKSON ; SARAH WILSONA.** Cognition-related brain networks underpin the symptoms of unipolar depression: Evidence from a systematic review. *Neuroscience and Biobehavioral Reviews,* 2016, vol. 61, 53-65

- **JUN-ICHIRO HIRAYAMA ; AAPO HYVARINEN ; MOTOAKI KAWANABE.** SPLICE: Fully Tractable Hierarchical Extension of ICA with Pooling. *ICML,* 2017, 1491-1500